# EUROPEAN PATENT SPECIFICATION

(11) **EP 1 790 303 B1**
(45) Date of publication and mention of the grant of the patent: **03.05.2017**
(21) Application number: 05745632.9
(22) Date of filing: 31.05.2005
(51) Int. Cl.: A61B 17/32, A61B 18/00

(54) **ULTRASONIC TREATMENT APPARATUS, AND PROBE,TREATMENT PORTION AND THICK PORTION FOR ULTRASONIC TREATMENT APPARATUS**
ULTRASCHALLBEHANDLUNGSVORRICHTUNG, SONDE, BEHANDLUNGSABSCHNITT UND DICKER ABSCHNITT FÜR EINE SOLCHE VORRICHTUNG
INSTRUMENT DE TRAITEMENT ULTRASONORE, SONDE, SECTION DE TRAITEMENT ET SECTION GROSSE POUR LEDIT INSTRUMENT ULTRASONORE

(30) Priority: 14.09.2004 JP 2004267074
(43) Date of publication of application: 30.05.2007
(73) Proprietor: Olympus Corporation, Tokyo 192-8507 (JP)
(72) Inventor: YOSHIMINE, Hideto, Hachioji-shi, Tokyo 192-8512 (JP); YAMADA, Norihiro, Hachioji-shi, Tokyo 195-8512 (JP)
(74) Representative: von Hellfeld, Axel
(86) International application number: PCT/JP2005/009979
(87) International publication number: WO 2006/030563

(56) References cited:
- EP-A- 1 199 042
- EP-A- 1 396 232
- JP-A- 5 115 490
- JP-A- 8 503 621
- JP-A- 2002 035 001
- US-A- 3 433 226
- US-A- 3 636 943
- US-A- 3 896 811
- US-A- 4 838 853
- US-A- 5 324 255
- US-A- 5 324 299
- US-A- 5 906 628

## Description

### Technical Field

The present invention relates to an ultrasonic treatment apparatus which applies ultrasonic vibrations to a treatment target to perform a treatment.

### Background Art

There has been conventionally used an ultrasonic treatment apparatus which applies ultrasonic vibrations to a living tissue to perform a treatment. In such an ultrasonic treatment apparatus, a probe which utilizes ultrasonic vibrations transmitted from an ultrasonic transducer to perform a treatment is connected with the ultrasonic transducer which generates ultrasonic vibrations. An example of such a probe is disclosed in U.S. Patent No. 5,324,299.

The probe disclosed in U.S. Patent No. 5,324,299 is a surgical blade which is used to make an incision in a living tissue. This surgical blade has an elongated vibration transmitting member which transmits ultrasonic vibrations. A proximal end portion of this vibration transmitting member is connected with an ultrasonic transducer. On the other hand, an distal end portion of the vibration transmitting member has a long plate-like shape and forms a blade portion which makes an incision in a living tissue. A concave hook portion which holds a living tissue is formed in one side end surface portion of this blade portion.

In case of giving a treatment to a living tissue, the proximal end portion of the vibration transmitting member is connected with the ultrasonic transducer. Further, a bulge portion of the living tissue is fitted in the hook portion to hold the living tissue. Then, ultrasonic vibrations are generated by the ultrasonic transducer, and the generated ultrasonic vibrations are transmitted from the proximal end side to the distal end side by the vibration transmitting member, thereby causing the blade portion to ultrasonic vibrate. Furthermore, the ultrasonic vibrations are given to the living tissue by the blade portion to make an incision therein while holding the living tissue in the hook portion. Moreover, there is also performed a treatment which emulsifies and fractures a tissue by utilizing ultrasonic vibrations of the distal end of the treatment portion. Incidentally, in case of performing such a treatment by utilizing ultrasonic vibrations, a transmission speed of ultrasonic vibrations varies depending on properties of a tissue, and there is an advantage that the treatment can be given to tissues alone which surround a blood vessel.

Document US 5,906,628 concerns an ultrasonic treatment instrument having a hook-shaped treatment section for performing an ultrasonic treatment of organic tissue and a reception member. Between the hook-shaped treatment section and the reception member, the organic tissue can be held.

### Disclosure of Invention

The ultrasonic treatment apparatus is employed in
various kinds of manners in accordance with treatment operations to be implemented. For example, a treatment such as coagulation is often performed by pressing a distal end portion of the probe against a living tissue and giving ultrasonic vibrations from the distal end surface portion to the living tissue. If such an operation is performed with a probe such as a surgical blade disclosed in U.S. Patent No. 5,324,299 , the living tissue slips due to the ultrasonic vibrations of the probe and it is difficult to certainly perform the treatment.

The present invention has been accomplished to solve the above problem. The object of the present invention is to provide an ultrasonic treatment apparatus capable of preventing the slipping of a treatment target to certainly perform the treatment.

Some or all of the above problems can be overcome by a treatment portion for an ultrasonic treatment apparatus having the features of claim 1.

An aspect of the present invention is an ultrasonic treatment apparatus characterized by including: an ultrasonic transducer which generates ultrasonic vibrations; an elongated vibration transmitting member whose proximal end portion is connected with the ultrasonic transducer and which transmits the ultrasonic vibrations from the proximal end portion to an distal end portion; and a treatment portion which is provided at the distal end portion of the vibration transmitting member and applies the ultrasonic vibrations to a treatment target, wherein
the treatment portion includes an distal end surface portion, and the distal end surface portion includes a holding portion configured to hold the treatment target.

In this aspect, the treatment target is held by the holding portion of the distal end surface portion of the treatment portion while ultrasonic vibrations are generated by the ultrasonic transducer, the generated ultrasonic vibrations are transmitted to the treatment portion by the vibration transmitting member, and the distal end surface portion of the treatment portion is pressed against the treatment target, to give the ultrasonic vibrations thereto. The treatment target is thereby treated.

Preferably, the holding portion includes at least one concave portion formed on the distal end surface portion.

In this preferred aspect, the treatment target is fitted in the concave portion of the distal end surface portion, and the treatment target is thereby held.

Preferably, the holding portion includes at least one convex portion formed on the distal end surface portion.

In this preferred aspect, the treatment target is engaged with a side surface portion of the convex portion of the distal end surface portion, and the treatment target is thereby held.

Preferably, the holding portion includes at least one rough surface portion which is formed on the distal end surface portion and holds the treatment target by friction.

In this preferred aspect, the treatment target is held by friction between the rough surface portion of the distal end surface portion and the treatment target.

Preferably, the ultrasonic treatment apparatus further includes an indicator indicating characteristics of the holding portion.

In this preferred aspect, the characteristics of the holding portion are identified by means of the indicator.

Preferably, the characteristics of the holding portion include an arrangement or a type of the holding portion.

In this preferred aspect, the arrangement or the type of the holding portion is identified by means of the indicator.

Preferably, the treatment portion and the vibration transmitting member include peripheral surface portions, and the indicator is formed on the peripheral surface portion of the treatment portion or the vibration transmitting member.

In this preferred aspect, the characteristics of the holding portion of the distal end surface portion of the treatment portion are identified by means of the indicator formed on the peripheral portion of the treatment portion or vibration transmitting member.

Preferably, the vibration transmitting member and the treatment portion includes a channel which penetrates the vibration transmitting member and the treatment portion from a proximal end side toward an distal end side and includes an opening portion on the distal end surface portion.

In this preferred aspect, solution sending, suction, etc. are executed at the opening portion of the distal end surface portion via the channel.

Another aspect of the present invention is a probe used for the above ultrasonic treatment apparatus, including the vibration transmitting member and the treatment portion of the above ultrasonic treatment apparatus.

Another aspect of the present invention is an ultrasonic treatment apparatus characterized by including: an ultrasonic transducer which generates ultrasonic vibrations; and a treatment portion whose proximal end side is connected with the ultrasonic transducer, and which transmits the ultrasonic vibrations generated by the ultrasonic transducer and applies the ultrasonic vibrations to a treatment target, wherein the treatment portion includes an distal end surface portion provided on an distal end side, and the distal end surface portion includes a holding portion which is configured to hold the treatment target.

In this aspect, the treatment target is held by the holding portion of the distal end surface portion of the treatment portion while ultrasonic vibrations are generated by the ultrasonic transducer, the generated ultrasonic vibrations are transmitted by the treatment portion, and the distal end surface portion is pressed against the treatment target, to give the ultrasonic vibrations thereto. The treatment target is thereby treated.

Preferably, the treatment portion includes a thin portion extending from the proximal end side toward the distal end side and a thick portion provided at an distal end portion of the thin portion, and an external diameter of at least a part of cross section of the thick portion perpendicular to a longitudinal axis of the thin portion is larger than an external diameter of at least a part of a cross section of the thin portion perpendicular to the longitudinal axis of the thin portion.

In this preferred aspect, the treatment target is treated by the thick portion which is provided at the distal end portion of the thin portion and which has a greater external diameter than an external diameter of the thin portion.

Preferably, the treatment portion includes a knife-shaped edge portion provided to the thin portion or between the thin portion and the thick portion.

In this preferred aspect, the incision treatment is executed on the treatment target by means of the knife-shaped edge portion.

Preferably, the treatment portion includes a channel which penetrates the treatment portion from the proximal end side toward the distal end side and includes an opening portion on the distal end surface portion.

In this preferred aspect, solution sending, suction, etc. are executed at the opening portion of the distal end surface portion via the channel.

Preferably, a diameter of the channel in the opening portion is smaller than that of the channel on the proximal end side of the opening portion.

In this preferred aspect, the treatment target is treated by portions other than the opening portion, at the distal end surface portion.

Preferably, the treatment portion is detachably provided to the vibration transmitting member.

In this preferred aspect, the treatment portion is exchangeable.

Preferably, the thick portion is detachably provided to the thin portion.
In this preferred aspect, the thick portion is exchangeable.

Preferably, the distal end surface portion is formed of ceramics.

In this preferred aspect, the distal end surface portion is formed of ceramic having high durability.

Another aspect of the present invention is a detachable treatment portion used for the ultrasonic treatment apparatus.

Another aspect of the present invention is a detachable thick portion used for the ultrasonic treatment apparatus.

According to the present invention, it is possible to prevent the slipping of the treatment target to certainly perform the treatment.

### Brief Description of Drawings

FIG. 1A is a longitudinal cross-sectional view showing an ultrasonic treatment apparatus according to a first embodiment of the present invention.
FIG. 1B is a perspective view showing a treatment portion of the ultrasonic treatment apparatus according to the first embodiment of the present invention.
FIG. 2 is a perspective view showing a treatment portion of an ultrasonic treatment apparatus according to a first modification of the first embodiment of the present invention.
FIG. 3 is a perspective view showing a treatment portion of an ultrasonic treatment apparatus according to a second embodiment of the present invention.
FIG. 4 is a perspective view showing a treatment portion of an ultrasonic treatment apparatus according to a third embodiment of the present invention.
FIG. 5 is a perspective view showing a treatment portion of an ultrasonic treatment apparatus according to a fourth embodiment of the present invention.
FIG. 6 is a perspective view showing a treatment portion of an ultrasonic treatment apparatus according to a fifth embodiment of the present invention.
FIG. 7 is a perspective view showing a treatment portion of an ultrasonic treatment apparatus according to a sixth embodiment of the present invention.
FIG. 8 is a perspective view showing a treatment portion of an ultrasonic treatment apparatus according to a seventh embodiment of the present invention.
FIG. 9A is a perspective view showing a treatment portion of an ultrasonic treatment apparatus according to a first modification of the seventh embodiment of the present invention.
FIG. 9B is a perspective view showing a treatment portion of another ultrasonic treatment apparatus according to the first modification of the seventh embodiment of the present invention.
FIG. 9C is a perspective view showing a treatment portion of an ultrasonic treatment apparatus according to a second modification of the seventh embodiment of the present invention.
FIG. 10 is a perspective view showing a treatment portion and an ultrasonic transducer of an ultrasonic treatment apparatus according to an eighth embodiment of the present invention.
FIG. 11 is a perspective view showing a treatment portion and an ultrasonic transducer of an ultrasonic treatment apparatus according to a ninth embodiment of the present invention.
FIG. 12A is a perspective view showing the treatment portion of the ultrasonic treatment apparatus according to the ninth embodiment of the present invention.
FIG. 12B is a top view showing the treatment portion of the ultrasonic treatment apparatus according to the ninth embodiment of the present invention.
FIG. 12C is a front view showing the treatment portion of the ultrasonic treatment apparatus according to the ninth embodiment of the present invention.
FIG. 13A is a top view showing a treatment portion of an ultrasonic treatment apparatus according to a first modification of the ninth embodiment of the present invention.
FIG. 13B is a front view showing the treatment portion of the ultrasonic treatment apparatus according to the first modification of the ninth embodiment of the present invention.
FIG. 14A is a top view showing a treatment portion of an ultrasonic treatment apparatus according to a second modification of the ninth embodiment of the present invention.
FIG. 14B is a front view showing the treatment portion of the ultrasonic treatment apparatus according to the second modification of the ninth embodiment of the present invention.
FIG. 15A is a top view showing a treatment portion of an ultrasonic treatment apparatus according to a third modification of the ninth embodiment of the present invention.
FIG. 15B is a front view showing the treatment portion of the ultrasonic treatment apparatus according to the third modification of the ninth embodiment of the present invention.
FIG. 16A is a top view showing a treatment portion of an ultrasonic treatment apparatus according to a fourth modification of the ninth embodiment of the present invention.
FIG. 16B is a front view showing the treatment portion of the ultrasonic treatment apparatus according to the fourth modification of the ninth embodiment of the present invention.
FIG. 17A is a top view showing a treatment portion of an ultrasonic treatment apparatus according to a fifth modification of the ninth embodiment of the present invention.
FIG. 17B is a front view showing the treatment portion of the ultrasonic treatment apparatus according to a fifth modification of the ninth embodiment of the present invention.
FIG. 18A is a top view showing a treatment portion of an ultrasonic treatment apparatus according to a sixth modification of the ninth embodiment of the present invention.
FIG. 18B is a front view showing the treatment portion of the ultrasonic treatment apparatus according to the sixth modification of the ninth embodiment of the present invention.
FIG. 19A is a top view showing a treatment portion of an ultrasonic treatment apparatus according to a seventh modification of the ninth embodiment of the present invention.
FIG. 19B is a front view showing the treatment portion of the ultrasonic treatment apparatus according to the seventh modification of the ninth embodiment of the present invention.
FIG. 20A is a top view showing a treatment portion of an ultrasonic treatment apparatus according to an eighth modification of the ninth embodiment of the present invention.
FIG. 20B is a front view showing the treatment portion of the ultrasonic treatment apparatus according to the eighth modification of the ninth embodiment of the present invention.
FIG. 21A is a top view showing a treatment portion of an ultrasonic treatment apparatus according to a ninth modification of the ninth embodiment of the present invention.
FIG. 21B is a front view showing the treatment portion of the ultrasonic treatment apparatus according to the ninth modification of the ninth embodiment of the present invention.
FIG. 22A is a top view showing a treatment portion of an ultrasonic treatment apparatus according to a tenth modification of the ninth embodiment of the present invention.
FIG. 22B is a front view showing the treatment portion of the ultrasonic treatment apparatus according to the tenth modification of the ninth embodiment of the present invention.
FIG. 23A is a top view showing a treatment portion of an ultrasonic treatment apparatus according to an eleventh modification of the ninth embodiment of the present invention.
FIG. 23B is a front view showing the treatment portion of the ultrasonic treatment apparatus according to the eleventh modification of the ninth embodiment of the present invention.
FIG. 24A is a top view showing a treatment portion of an ultrasonic treatment apparatus according to a twelfth modification of the ninth embodiment of the present invention.
FIG. 24B is a front view showing the treatment portion of the ultrasonic treatment apparatus according to the twelfth modification of the ninth embodiment of the present invention.
FIG. 25A is a perspective view showing a treatment portion of an ultrasonic treatment apparatus according to a thirteenth modification of the ninth embodiment of the present invention.
FIG. 25B is a top view showing the treatment portion of the ultrasonic treatment apparatus according to the thirteenth modification of the ninth embodiment of the present invention.
FIG. 25C is a front view showing the treatment portion of the ultrasonic treatment apparatus according to the thirteenth modification of the ninth embodiment of the present invention.
FIG. 26A is a perspective view showing treatment portion of an ultrasonic treatment apparatus according to a fourteenth modification of the ninth embodiment of the present invention.
FIG. 26B is a top view showing the treatment portion of the ultrasonic treatment apparatus according to the fourteenth modification of the ninth embodiment of the present invention.
FIG. 26C is a front view showing the treatment portion of the ultrasonic treatment apparatus according to the fourteenth modification of the ninth embodiment of the present invention.
FIG. 27 is a top view showing a treatment portion of an ultrasonic treatment apparatus according to a fifteenth modification of the ninth embodiment of the present invention.
FIG. 28A is a perspective view showing a treatment portion of an ultrasonic treatment apparatus according to a tenth embodiment of the present invention.
FIG. 28B is a top view showing the treatment portion of the ultrasonic treatment apparatus according to the tenth embodiment of the present invention.
FIG. 29A is a perspective view showing a treatment portion of an ultrasonic treatment apparatus according to a first modification of the tenth embodiment of the present invention.
FIG. 29B is a top view showing the treatment portion of the ultrasonic treatment apparatus according to the first modification of the tenth embodiment of the present invention.
FIG. 29C is a lateral cross-sectional view of the treatment portion of the ultrasonic treatment apparatus according to the first modification of the tenth embodiment of the present invention taken along a line XXIXC-XXIXC in FIG. 29B.
FIG. 30A is a perspective view showing a treatment portion of an ultrasonic treatment apparatus according to a second modification of the tenth embodiment of the present invention.
FIG. 30B is a top view showing the treatment portion of the ultrasonic treatment apparatus according to the second modification of the tenth embodiment of the present invention.
FIG. 30C is a lateral cross-sectional view showing the treatment portion of the ultrasonic treatment apparatus according to the second modification of the tenth embodiment of the present invention taken along a line XXXC-XXXC in FIG. 30B.
FIG. 31 is a perspective view showing a treatment portion of an ultrasonic treatment apparatus according to an eleventh embodiment of the present invention.
FIG. 32 is a longitudinal cross-sectional view showing a treatment portion of an ultrasonic treatment apparatus according to a twelfth embodiment of the present invention.
FIG. 33A is a perspective view showing an ultrasonic treatment apparatus according to a thirteenth embodiment of the present invention.
FIG. 33B is a perspective view showing the treatment portion of the ultrasonic treatment apparatus according to the thirteenth embodiment of the present invention.
FIG. 34 is a longitudinal cross-sectional view showing a treatment portion of an ultrasonic treatment apparatus according to a fourteenth embodiment of the present invention.
FIG. 35 is an exploded view showing an ultrasonic treatment apparatus according to a fifteenth embodiment of the present invention.
FIG. 36 is an exploded view showing an ultrasonic treatment apparatus according to a modification of the fifteenth embodiment of the present invention.
FIG. 37 is an exploded view showing a treatment portion of an ultrasonic treatment apparatus according to a sixteenth embodiment of the present invention.
FIG. 38 is a perspective view showing a treatment portion of an ultrasonic treatment apparatus according to a modification of the sixteenth embodiment of the present invention.

### Best Mode for Carrying Out the Invention

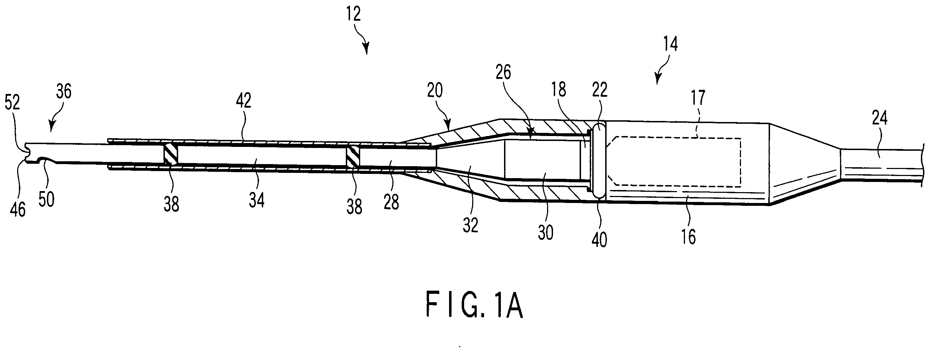

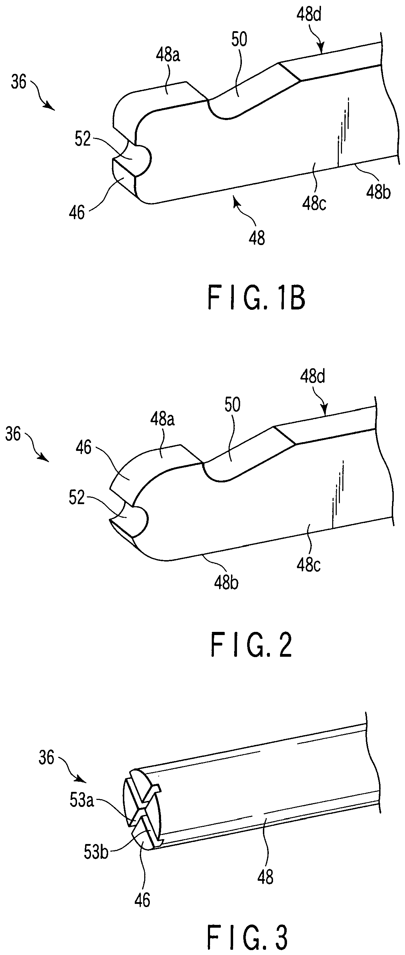

A first embodiment according to the present invention will now be described with reference to FIGS. 1A and 1B. FIG. 1A shows an entire schematic configuration of an ultrasonic treatment apparatus 12 according to this embodiment. This ultrasonic treatment apparatus 12 has a grip portion 14 which is gripped by an operator. An ultrasonic transducer 17 which generates ultrasonic vibrations is accommodated in a main body portion 16 of this grip portion 14. In this embodiment, a bolted Langevin type transducer (BLT) is used as the ultrasonic transducer 17. It is to be noted that one having a function of converting electrical vibrations into mechanical vibrations can be used as the ultrasonic transducer 17, and the ultrasonic transducer 17 may be a laminated piezoelectric transducer, a magnetostrictive transducer, an electrostrictive polymer transducer, an air gap type electrostatic actuator or the like.

An output end 18 which outputs ultrasonic vibrations is arranged on an distal end side of this ultrasonic transducer 17. This output end 18 has a substantially cylindrical shape and protrudes from an distal end surface of the main body portion 16. Further, a non-illustrated male screw portion is provided at a central part of the distal end surface of the output end 18 to protrude toward the distal end side. An attachment/detachment portion 22 which is used to attach/detach a later-described sheath 20 is arranged at a rim portion of the distal end surface of the main body portion 16. This attachment/detachment portion 22 is formed of a C-ring, an O-ring or the like. On the other hand, a cord 24 through which an electrical signal used to drive the ultrasonic transducer 17 is extended from a proximal end portion of the main body portion 16. An extended end portion of this cord 24 is connected with a non-illustrated drive power supply.

A probe 26 which utilizes the ultrasonic vibrations transmitted from the ultrasonic transducer 17 to perform a treatment is connected with the ultrasonic transducer 17. This probe 26 has an elongated cylindrical vibration transmitting member 28 which transmits the ultrasonic vibrations. A thick portion 30 having substantially the same external diameter as that of the output end 18 of the ultrasonic transducer 17 is formed at a proximal end portion of this vibration transmitting member 28. A non-illustrated female screw portion which extends in an axial direction of the vibration transmitting member 28 is bored in a proximal end surface of this thick portion 30. This female screw portion corresponds to the male screw portion of the output end 18, and the vibration transmitting member 28 is connected with the ultrasonic transducer 17 by screwing the female screw portion with respect to the male screw portion.

On the other hand, a tapered portion 32 whose external diameter is reduced from a proximal end side toward an distal end side is coupled with the distal end side of the thick portion 30. This tapered portion 32 is used to amplify the ultrasonic vibrations transmitted through the vibration transmitting member 28. Furthermore, an elongated thin portion 34 is coupled with the distal end side of the tapered portion 32. In this embodiment, an distal end portion of the thin portion 34 has a long plate-like shape, and a treatment portion 36 which applies the ultrasonic vibrations to a living tissue as a treatment target is formed at this portion. That is, in this embodiment, the treatment portion 36 is integrally formed with the vibration transmitting member 28.

In this embodiment, the ultrasonic transducer 17 and the vibration transmitting member 28 are configured to perform longitudinal vibrations. Moreover, an entire length when the ultrasonic transducer 17 is connected with the vibration transmitting member 28 is a length which is an integral multiple of a half-wavelength of the ultrasonic vibrations in such a manner that the treatment portion 36 is placed at an antinode position of the ultrasonic vibrations. Additionally, an annular support member 38 is externally arranged at a node position of an ultrasonic vibration on the thin portion 34 of the vibration transmitting member 28. This support member 38 has elasticity, and is formed of, e.g., silicone rubber. Further, the support member 38 is held on an inner peripheral surface of the later-described sheath 20, and supports the vibration transmitting member 28.

The cylindrical sheath 20 is externally provided on the vibration transmitting member 28. An attachment/detachment portion acceptor 40 which is attached/detached with respect to the attachment/detachment portion 22 of the ultrasonic transducer 17 is formed at a proximal end portion of the sheath 20. When the attachment/detachment portion acceptor 40 is fitted on the attachment/detachment portion 22, the sheath 20 is attached with respect to the ultrasonic transducer 17. On the other hand, an distal end side of the sheath 20 is formed of an insertion pipe 42, and the thin portion 34 of the vibration transmitting member 28 is inserted into this insertion pipe 42. An outer peripheral portion of the support member 38 mounted on the thin portion 34 is in contact with and held on an inner peripheral surface of the insertion pipe 42. Furthermore, the treatment portion 36 of the distal end portion of the vibration transmitting member 28 protrudes from an distal end opening of the sheath 20.

As shown in FIG. 1B, the treatment portion 36 has a long plate-like shape in this embodiment. That is, the treatment portion 36 has a substantially rectangular distal end surface portion 46 and a peripheral surface portion 48, and the peripheral surface portion 48 has first and second narrow side surface portions 48a and 48b each having a narrow width and first and second wide side surface portions 48c and 48d each having a wide width. A hook portion 50 which is a concave portion having a hook-like shape is formed in the first narrow side surface portion 48a. Moreover, a concave portion 52 which extends across the distal end surface portion 46 in the width direction of the distal end surface portion 46 is formed in the distal end surface portion 46. These hook portion 50 and concave portion 52 hold a fitted living tissue, and the concave portion 52 forms a holding portion.

Again referring to FIG. 1A, a pressure wave is emitted from the distal end surface portion 46 of the treatment portion 36 toward the living tissue pressed against the distal end surface portion 46 by longitudinal vibrations of the vibration transmitting member 28. The pressure wave emitted from the distal end surface portion 46 is converged toward the center of a curvature radius of the concave portion 52 by a function of the concave portion 52. That is, the concave portion 52 has a function of converging the ultrasonic vibrations given to the living tissue in front of the distal end surface portion 46.

A function of the ultrasonic treatment apparatus 12 according to this embodiment will now be described. In case of using the ultrasonic treatment apparatus 12, the female screw portion of the thick portion 30 of the vibration transmitting member 28 is screwed with respect to the male screw portion of the output end 18 of the ultrasonic transducer 17 so that the vibration transmitting member 28 is connected with the ultrasonic transducer 17. Moreover, the sheath 20 is externally provided on the vibration transmitting member 28, and the attachment/detachment portion acceptor 40 of the sheath 20 is fitted on the attachment/detachment portion 22 of the ultrasonic transducer 17, whereby the sheath 20 is attached on the ultrasonic transducer 17. Additionally, the cord 24 of the ultrasonic transducer 17 is connected with the drive power supply.

Further, the grip portion 14 is gripped and operated to approach the treatment portion 36 toward a target part of a living tissue. Here, the living tissue has a part where a bulge portion is formed, e.g., a bulge part where a blood vessel runs on a surface part. In case of giving a treatment to such a part, the distal end surface portion 46 of the treatment portion 36 is pressed against the living tissue, and the bulge portion of the living tissue is fitted in the concave portion 52 of the distal end surface portion 46 so that the living tissue is held in the concave portion 52.

In this state, an electrical signal is input to the ultrasonic transducer 17 from the drive power supply to generate ultrasonic vibrations, and the generated ultrasonic vibrations are transmitted to the treatment portion 36 by the vibration transmitting member 28. The ultrasonic vibrations are given to the held living tissue from the distal end surface portion 46 while holding the living tissue in the concave portion 52, whereby a treatment such as coagulation is given to the living tissue. It is to be noted that the ultrasonic vibrations given to the living tissue from the distal end surface portion 46 are converged in front of the distal end surface portion 46 by the function of the concave portion 52. If necessary, the ultrasonic vibrations are given to the held living tissue from the first narrow side surface portion 48a while holding the living tissue by the hook portion 50, thereby giving a treatment such as an incision to the living tissue.

Therefore, the ultrasonic treatment apparatus 12 according to this embodiment includes the following effect. In this embodiment, the distal end surface portion 46 of the treatment portion 36 is pressed against the living tissue, the bulge portion of the living tissue is fitted in the concave portion 52 of the distal end surface portion 46 to hold the living tissue in the concave portion 52, and the ultrasonic vibrations are given to the held living tissue, thereby giving a treatment. Therefore, the living tissue is prevented from slipping during a treatment, and the treatment can be assuredly given to a target position.

It is to be noted that the vibration transmitting member 28 and the treatment portion 36 are integrally formed in this embodiment, but the treatment portion 36 may be separately provided at the distal end portion of the vibration transmitting member 28.

FIG. 2 shows a modification of the first embodiment according to the present invention. Like reference numbers denote structures having the same functions as those in the first embodiment, thereby eliminating their explanation. In this modification, the first and second narrow side surface portions 48a and 48b are smoothly connected with the distal end surface portion 46 in the treatment portion 36 according to the first embodiment.

FIG. 3 shows a second embodiment according to the present invention. Like reference numbers denote structures having the same functions as those in the first embodiment, thereby eliminating their explanation. In this embodiment, a treatment portion 36 has a cylindrical shape and has a circular distal end surface portion 46. First and second groove-shaped concave portions 53a and 53b which extend in a diametric direction of the distal end surface portion 46 are formed in this distal end surface portion 46. These first and second concave portions 53a and 53b respectively form a holding portion which holds a living tissue. It is to be noted that the first and second concave portions 53a and 53b are substantially orthogonal to each other.

A function of an ultrasonic treatment apparatus 12 according to this embodiment will now be described. A living tissue has a part where an elongated bulge portion is formed, e.g., a part where a blood vessel runs. In case of giving a treatment to such a part, after a treatment portion 36 is moved close to a target part, a grip portion 14 is operated to rotate a vibration transmitting member 28 about its central axis so that a direction (a longitudinal direction) of the first or second groove-shaped concave portion 53a or 53b is matched with a direction (a longitudinal direction) of an elongated bulge portion of the living tissue. Then, the distal end surface portion 46 is pressed against the living tissue, and the bulge portion of the living tissue is fitted and hold in the first or second concave portion 53a or 53b. Incidentally, of the first concave portion 53a and the second concave portion 53b, the concave portion 52 which can be matched with the direction of the bulge portion with a small amount of rotational operation is used for holding the living tissue.

Therefore, the ultrasonic treatment apparatus 12 according to this embodiment includes the following effect. Since each of the first and second concave portions 53a and 53b has a groove-like shape, it is suitable to hold a living tissue having an elongated bulge portion formed thereon.

Moreover, in case of holding a living tissue having an elongated bulge portion formed thereon, the vibration transmitting member 28 must be rotated about its central axis to match a direction of the first or second concave portion 53a or 53b with a direction of the bulge portion. Here, the first and second concave portions 53a and 53b are substantially orthogonal to each other. Of the first concave portion 53a and the second concave portion 53b, either the concave portion 53a or 53b whose direction can be matched with the direction of the bulge portion with a small amount of rotational operation is used to hold the living tissue. Therefore, the operation of matching the directions is facilitated.

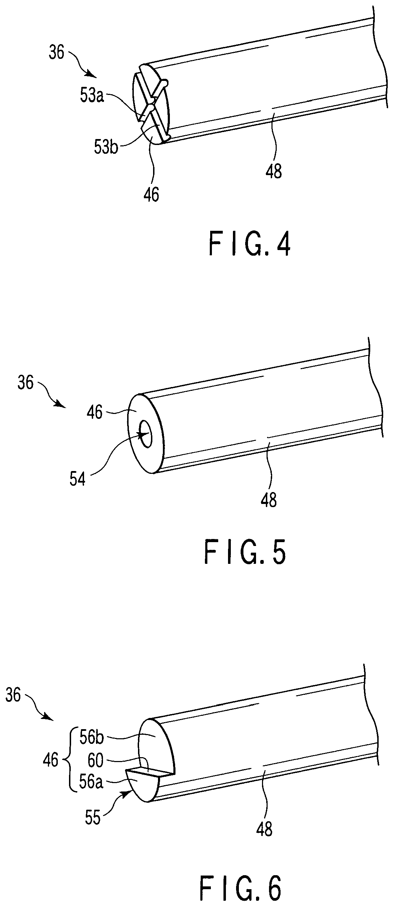

FIG. 4 shows a third embodiment according to the present invention. Like reference numbers denote structures having the same functions as those in the second embodiment, thereby eliminating their explanation. In this embodiment, a width of a first concave portion 53a is larger than a width of a second concave portion 53b. A living tissue has a part where elongated bulge portions with various widths are formed, e.g., a part where a plurality of blood vessels having different thicknesses run. In case of giving a treatment to such a part, the first concave portion 53a is used with respect to a wide bulge portion to hold a living tissue, and the second concave portion 53b is used with respect to a narrow bulge portion to hold the living tissue. In this embodiment, the wide first concave portion 53a and the narrow second concave portion 53b are selectively used in accordance with a width of an elongated bulge portion of a living tissue in this manner. Therefore, it is possible to avoid a situation where a living tissue cannot be satisfactorily held because a width of a bulge portion is too large or too small for the widths of the concave portions 53a and 53b.

FIG. 5 shows a fourth embodiment. Like reference numbers denote structures having the same functions as those in the second embodiment, thereby eliminating their explanation. A semispherical concave portion 54 as a holding portion is formed in an distal end surface portion of a treatment portion 36 according to this embodiment. A living tissue has a part where a semispherical bulge portion is formed. In case of giving a treatment to such a part, the semispherical bulge portion is fitted in and held in the semispherical concave portion 54. The concave portion 54 as the holding portion according to this embodiment is suitable to hold a living tissue having a semispherical bulge portion formed thereon.

FIG. 6 shows a fifth embodiment according to the present invention. Like reference numbers denote structures having the same functions as those in the second embodiment, thereby eliminating their explanation. A convex portion 55 as a holding portion is formed on an distal end surface portion of a treatment portion 36 according to this embodiment. A top surface of this convex portion 55 forms a first pressing surface 56a, and a part of the distal end surface portion 46 where the convex portion 55 is not constituted forms a second pressing surface 56b. Additionally, a step 60 is formed of a side surface portion of the convex portion 55 between the first pressing surface 56a and the second pressing surface 56b. In this embodiment, the step 60 is linearly arranged across the distal end surface portion 46, and the first pressing surface 56a is smaller than the second pressing surface 56b.

A function of an ultrasonic treatment apparatus 12 according to this embodiment will now be described. A living tissue has a flat part which substantially has no bulge portion. In case of giving a treatment to such a part, the distal end surface portion 46 is pressed against a living tissue, and the first and second pressing surfaces 56a and 56b press the living tissue. As a result, the step 60 formed of the side surface portion of the convex portion 55 is brought into contact with and engaged with the living tissue, thereby holding the living tissue.

Therefore, the ultrasonic treatment apparatus 12 according to this embodiment includes the following effect. In this embodiment, the first and second pressing surfaces 56a and 56b press a living tissue, and the step 60 formed of the side surface portion of the convex portion 55 is brought into contact with and engaged with the living tissue, thereby holding the living tissues. Therefore, even a living tissue having no bulge portion can be assuredly held.

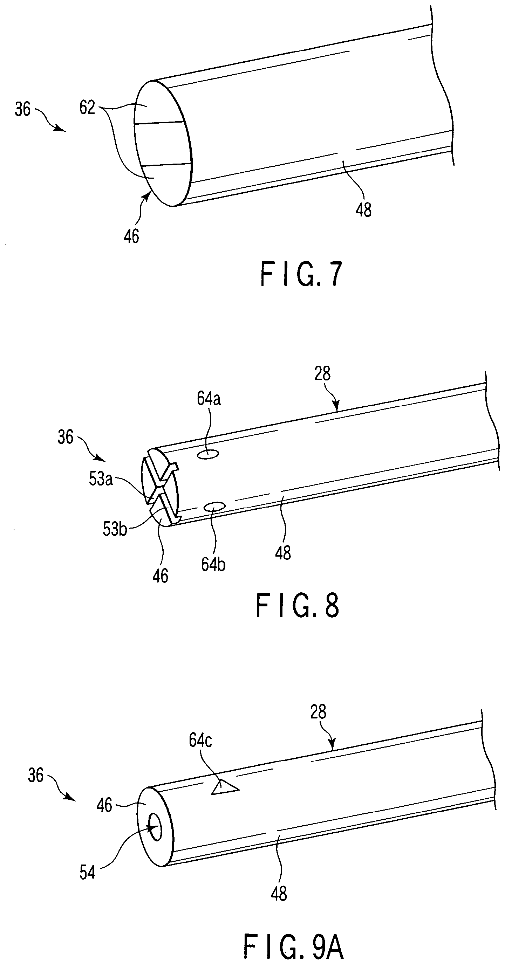

FIG. 7 shows a sixth embodiment according to the present invention. Like reference numbers denote structures having the same functions as those in the second embodiment, thereby eliminating their explanation. A rough surface portion 62 as a holding portion is formed on an distal end surface portion 46 of a treatment portion 36 according to this embodiment. In this embodiment, a surface in both side regions is rougher than a surface in a strip-like central region across the distal end surface portion 46, and both these side regions form the rough surface portion 62. In case of giving a treatment to a flat living tissue having substantially no bulge portion, the distal end surface portion 46 is pressed against the living tissue so that the rough surface portion 62 is brought into contact with the living tissue. As a result, the living tissue is held by friction between the rough surface portion 62 and the living tissue. As described above, in this embodiment, the rough surface portion 62 having the rough surface configured on the distal end surface portion 46 of the treatment portion 36 forms the holding portion which holds a living tissue. That is, a protruding shape is not formed on the distal end surface portion 46, and hence it is avoided that the protruding shape applies an excessive force to the living tissue.

FIG. 8 shows a seventh embodiment according to the present invention. Like reference numbers denote structures having the same functions as those in the second embodiment, thereby eliminating their explanation. A treatment portion 36 according to this embodiment has indicators 64a and 64b which are characteristic of a holding portion. In more detail, a configuration of the treatment portion 36 according to this embodiment is substantially the same as that of the treatment portion 36 according to the second embodiment. However, the first and second indicators 64a and 64b indicating arrangements of first and second concave portions 53a and 53b are arranged on a peripheral surface portion 48 of the treatment portion 36. Each of these first and second indicators 64a and 64b has an elliptic shape, and arranged to be aligned with each one distal end portion of the first and second groove-shaped concave portions 53a and 53b in an axial direction of a vibration transmitting member 28. The first and second indicators 64a and 64b can be used to guess arrangements of the first and second concave portions 53a and 53b.

A function of an ultrasonic treatment apparatus 12 according to this embodiment will now be described. In case of giving a treatment to a living tissue, an distal end surface portion 46 of the treatment portion 36 is moved closer to the living tissue. At this time, although it is difficult to visually confirm the distal end surface portion 46 of the treatment portion 36, visually confirming the peripheral surface portion 48 of the treatment portion 36 is relatively easy. Therefore, the first and second indicators 64a and 64b are used to take aim at arrangements of the first and second concave portions 53a and 53b, and the first and second concave portions 53a and 53b are aligned with respect to bulge portions of the living tissue.

Therefore, the ultrasonic treatment apparatus 12 according to this embodiment includes the following effect. The ultrasonic treatment apparatus 12 according to this embodiment has the indicators 64a and 64b which indicate characteristic of the holding portion. Therefore, for example, visually confirming the indicators 64a and 64b can indirectly recognize characteristics of the holding portion without directly visually confirming the holding portion.

Further, when moving the distal end surface portion 46 of the treatment portion 36 closer to a living tissue, it is hard to visually confirm the distal end surface portion 46 of the treatment portion 36. Since the indicators 64a and 64b according to this embodiment are arranged on the peripheral surface portion 48 of the treatment portion 36, visually confirming the indicators 64a and 64b on the peripheral surface portion 48 can recognize characteristics of the holding portion even if visually confirming the distal end surface portion 46 of the treatment portion 36 is difficult.

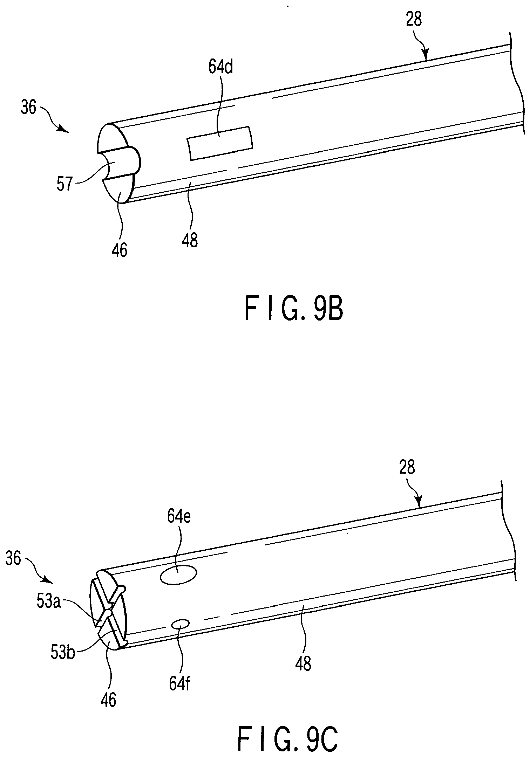

FIGS. 9A and 9B show a first modification of the seventh embodiment according to the present invention. Like reference numbers denote structures having the same functions as those in the seventh embodiment, thereby eliminating their explanation. As shown in FIGS. 9A and 9B, in this modification, triangular or rectangular indicators 64c and 64d are used in accordance with types of concave portions 54 and 57 as the holding portion. Therefore, visually confirming the indicators 64c and 64d can recognize a type of the holding portion.

FIG. 9C shows a second modification of the seventh embodiment according to the present invention. A configuration of a treatment portion 36 according to this embodiment is substantially the same as that of the treatment portion 36 according to the third embodiment. However, first and second indicators 64e and 64f indicative of arrangements and types of first and second concave portions 53a and 53b are arranged on a peripheral surface portion 48 of the treatment portion 36. The first indicator 64e has an elliptic shape, and is arranged to be aligned with one distal end portion of the first concave portion 53a having a wide groove-like shape in an axial direction of a vibration transmitting member 28. On the other hand, the second indicator 64f has an elliptic shape smaller than the first indicator 64e, and is arranged to be aligned with one distal end portion of the second concave portion 53b having a narrow groove-like shape in the axial direction of the vibration transmitting member 28. These first and second indicators 64e and 64f can be used to guess arrangements of the first and second concave portions 53a and 53b, and the type of the wide first concave portion 53a can be discriminated from the type of the narrow second concave portion 53b based on a difference in size between the first indicator 64e and the second indicator 64f.

Here, the indicators are used to indicate an arrangement of the holding portion in the seventh embodiment, they are used to indicate a type of the holding portion in the first modification, and they are used to indicate an arrangement and a type of the holding portion in the second modification. However, characteristics of the holding portion indicated by the indicators are not restricted thereto. The indicators may indicate a shape of each holding portion such as a quantity of irregularities, the number of concave portions as the holding portion, and others. Furthermore, although characteristics of each holding portion are identified based on a size of each indicator in the second modification, characteristics of each holding portion may be identified based on other shape characteristics such as a length, a depth or the like of each indicator. Moreover, characteristics of each holding portion may be identified based on the number, a color and others of the indicators. Additionally, characteristics of the holding portion can be directly grasped by configuring the holding portion to correspond to the indicators. For example, a semispherical concave indicator is used when the holding portion is a semispherical concave portion, a groove-shaped concave indicator is used when the holding portion is a groove-like concave portion, a deep concave indicator is used when the holding portion is a deep concave portion, and a shallow concave indicator is used when the holding portion is a shallow concave portion.

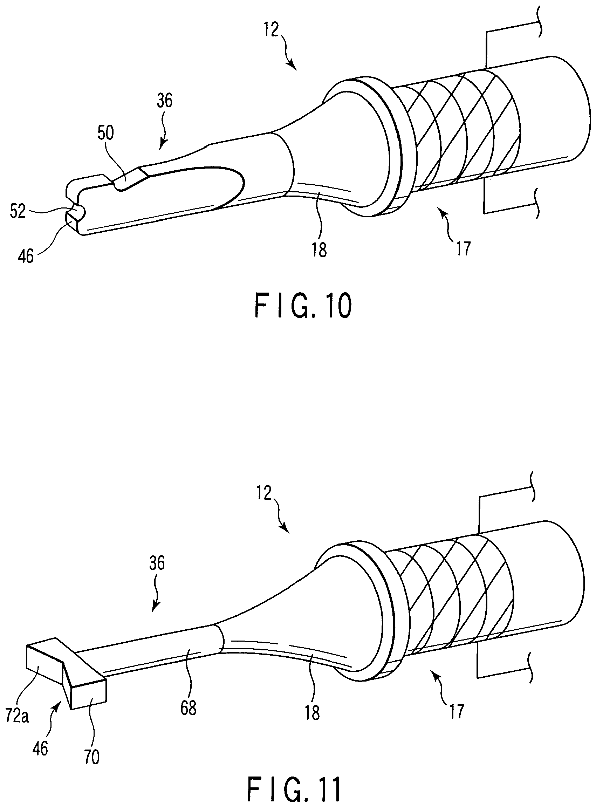

FIG. 10 shows an eighth embodiment according to the present invention. Like reference numbers denote structures having the same functions as those in the first embodiment, thereby eliminating their explanation. As shown in FIG. 10, an output end 18 of an ultrasonic transducer 17 has a horn-like shape whose diameter is reduced from a proximal end side toward an distal end side and by which ultrasonic vibrations are amplified. A treatment portion 36 is directly coupled with an distal end portion of this output end 18. In case of giving a treatment by using an ultrasonic treatment apparatus 12, the ultrasonic transducer 17 is used to generate ultrasonic vibrations, the generated ultrasonic vibrations are transmitted through the treatment portion 36, and an distal end surface portion 46 of the treatment portion 36 is pressed against a living tissue, thereby giving the ultrasonic vibrations to the living tissue. In this embodiment, the treatment portion 36 is directly coupled with the ultrasonic transducer 17, and a long vibration transmitting member 28 (see FIG. 1A) is not used, thereby reducing a size of the ultrasonic treatment apparatus 12. It is to be noted that the treatment portion 36 according to the first embodiment is used as the treatment portion 36 in this embodiment, but the treatment portion 36 according to each of the first to seventh embodiments and modifications thereof may be used.

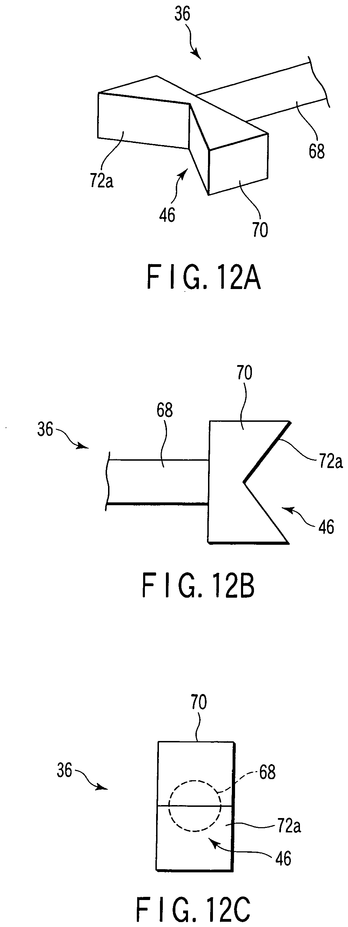

FIGS. 11 to 12C show a ninth embodiment according to the present invention. Like reference numbers denote structures having the same functions as those in the eighth embodiment, thereby eliminating their explanation.

As shown in FIG. 11, a treatment portion 36 has a thin portion 68 which extends from a proximal end side toward an distal end side and includes a longitudinal axis. A thick portion 70 is coupled with an distal end portion of this thin portion 68, and an external diameter of at least a part of a cross section of this thick portion 70 perpendicular to the longitudinal axis is larger than an external diameter of at least a part of a cross section of the thin portion 38 perpendicular to the longitudinal axis.

In more detail, as shown in FIGS. 11 to 12C, in this embodiment, a proximal end portion of the thin portion 68 having a substantially cylindrical shape is coupled with an distal end portion of an output end 18 of an ultrasonic transducer 17. The thick portion 70 having a substantially rectangular-parallelepiped shape is coupled with an distal end surface of this thin portion 68 in such a manner that its proximal end surface becomes substantially perpendicular to a central axis of the thin portion 68, and a cross section of the thick portion 70 perpendicular to the central axis has a substantially rectangular shape. Moreover, a long side and a short side of this rectangular shape are larger than a diameter of the thin portion 68. Additionally, a concave portion 72a as a holding portion is formed on the entire distal end surface of the thick portion 70, and this concave portion 72a has a triangular shape which opens toward the distal end side in a cross section parallel to both the long sides.

A function of the ultrasonic treatment apparatus 12 according to this embodiment will now be described. A description will be given as to a case where a tissue in which fibers are mixed is fractured by cavitation to perform a treatment, e.g., resection of a liver, abrasion of a mucous membrane of a stomach or a large intestine. An ultrasonic transducer 17 is used to generate ultrasonic vibrations, the generated ultrasonic vibrations are transmitted by the treatment portion 36 to cause the thick portion 70 to longitudinally vibrate, and the thick portion 70 in the vibrating state is pressed against a tissue. As a result, fibers of the tissue is collected in the concave portion 72a, the fibers are fractured by cavitation produced by the distal end surface portion 46, and the thick portion 70 dents the tissue. As a result, the fibers are caught by the proximal end surface of the thick portion 70, and the fibers are fractured by cavitation generated by the proximal end surface of the thick portion 70.

Therefore, the ultrasonic treatment apparatus 12 according to this embodiment includes the following effect. In the treatment portion 36 according to this embodiment, the external diameter of the thick portion 70 provided at the distal end portion of the thin portion 68 is larger than the external diameter of the thin portion 68. Therefore, in case of performing resection of a liver, abrasion of a mucous membrane of a stomach or a large intestine or the like, large cavitation can be generated by the thick portion 70, thereby improving a treatment capability of the ultrasonic treatment apparatus 12.

It is to be noted that the treatment portion 36 is directly connected with the ultrasonic transducer 17 in this embodiment, the configuration of the treatment portion 36 according to this embodiment can be also applied to an ultrasonic treatment apparatus 12 in which a treatment portion 36 is arranged at an distal end portion of a vibration transmitting member 28 like the first embodiment. This is also applied to all the following embodiments.

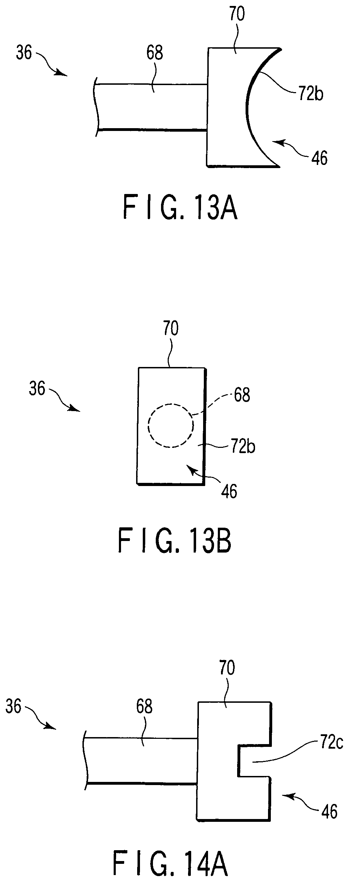

FIGS. 13A to 27 show first to fifteenth modifications of the ninth embodiment according to the present invention. Like reference numbers denote structures having the same functions as those in the ninth embodiment, thereby eliminating their explanation. These modifications provide an appropriate treatment portion corresponding to a treatment or a treatment target.

As shown in FIGS. 13A and 13B, a concave portion 72b as a holding portion is formed on an entire distal end surface in a thick portion 70 according to a first modification like the ninth embodiment, and this concave portion 72b has a semicircular shape which opens toward an distal end side in a cross section parallel to both long sides of the distal end surface.

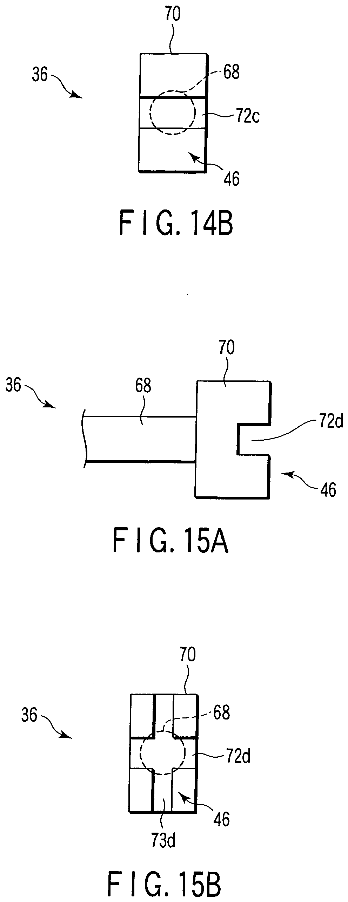

As shown in FIGS. 14A and 14B, on an distal end surface of a thick portion 70 according to a second modification, a groove-shaped concave portion 72c is provided to extend at a substantially central part of the distal end surface substantially parallel to short sides of the distal end surface. A cross section of this groove-shaped concave portion 72c perpendicular to a longitudinal direction thereof has a substantially square shape.

As shown in FIGS. 15A and 15B, first and second groove-shaped concave portions 72d and 73d are cruciately formed on an distal end surface of a thick portion 70 according to a third modification. That is, the first and second groove-shaped concave portions 72d and 73d are respectively arranged at a substantially central part of the distal end surface in substantially parallel to short sides and long sides of the distal end surface, and a cross section of each of the first and second groove-shaped concave portions 72d and 73d perpendicular to their longitudinal direction has a substantially square shape.

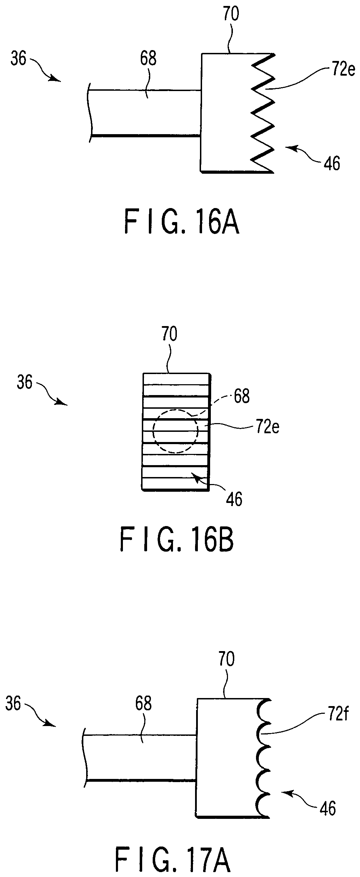

As shown in FIGS. 16A and 16B, a plurality of groove-shaped concave portions 72e substantially parallel to short sides of the distal end surface are closely aligned on an distal end surface of a thick portion 70 according to a fourth modification. A cross section of each of the plurality of groove-shaped concave portions 72e perpendicular to its longitudinal direction has a substantially triangular shape which opens toward the distal end side.

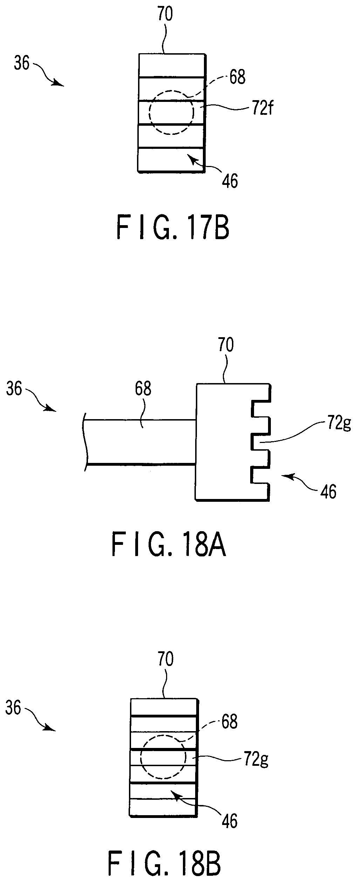

As shown in FIGS. 17A and 17B, a plurality of groove-shaped concave portions 72f substantially parallel to short sides of the distal end surface are closely aligned on an distal end surface of a thick portion 70 according to a fifth modification, like the fourth modification. A cross section of each of the plurality of groove-shaped concave portions 72f perpendicular to its longitudinal direction has a substantially semicircular shape which opens toward the distal end side.

As shown in FIGS. 18A and 18B, a plurality of groove-shaped concave portions 72g substantially parallel to short sides of the distal end surface are aligned to be apart from each other with a predetermined distance therebetween in a long side direction of an distal end surface on the distal end surface of a thick portion 70 according to a sixth modification. A cross section of each of the plurality of concave portions 72g perpendicular to its longitudinal direction has a substantially square shape.

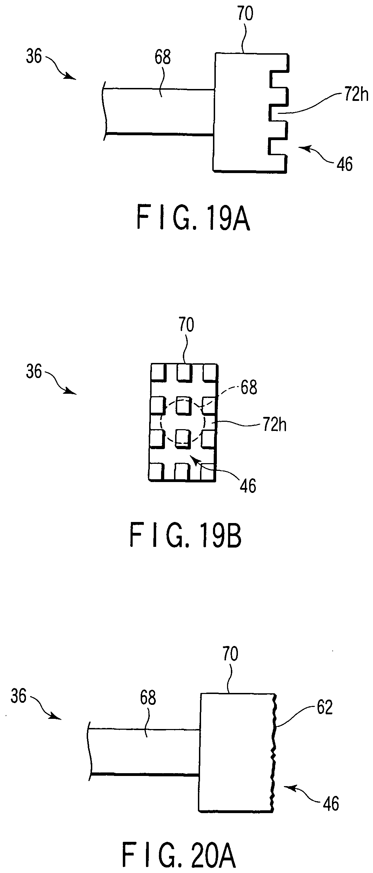

As shown in FIGS. 19A and 19B, a plurality of groove-shaped concave portions 72h are provided to extend in a grid pattern on an distal end surface of a thick portion 70 according to a seventh modification. That is, the plurality of groove-shaped concave portions 72h substantially parallel to short sides of the distal end surface are aligned to be apart from each other with a predetermined distance therebetween in a long side direction of the distal end surface, and the plurality of groove-shaped concave portions 72h substantially parallel to long sides of the distal end surface are aligned to be apart from each other with a predetermined distance therebetween in the short side direction of the distal end surface. A cross section of each of the plurality of groove-shaped concave portions 72h perpendicular to its longitudinal direction has a substantially square shape.

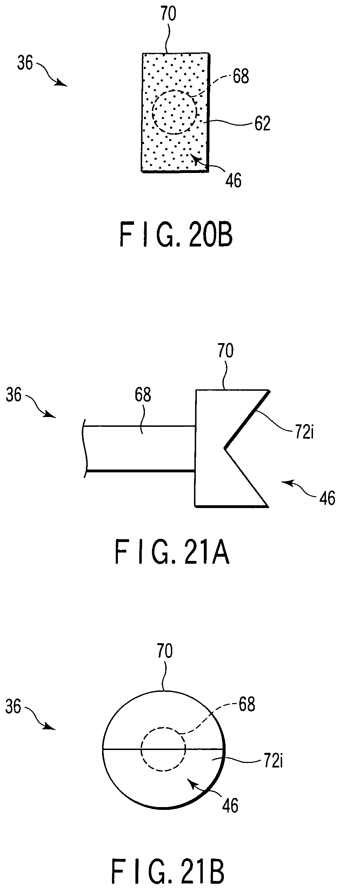

As shown in FIGS. 20A and 20B, in an eighth modification, a rough surface portion 62 as a holding portion like that in the sixth embodiment is arranged on an distal end surface portion 46 of the thick portion 70 according to the ninth embodiment.

As shown in FIGS. 21A and 21B, a thick portion 70 according to a ninth modification has a substantially short cylindrical shape whose central axis is coaxial with a thin portion 68. A concave portion 72i as a holding portion is formed on an entire distal end surface of the thick portion 70, and this concave portion 72i has a base bottom portion having a substantially linear shape which is substantially orthogonal to a central axis of the thin portion 68. Further, a cross section of the concave portion 72i perpendicular to its base bottom portion has a substantially triangular shape which opens toward an distal end side.

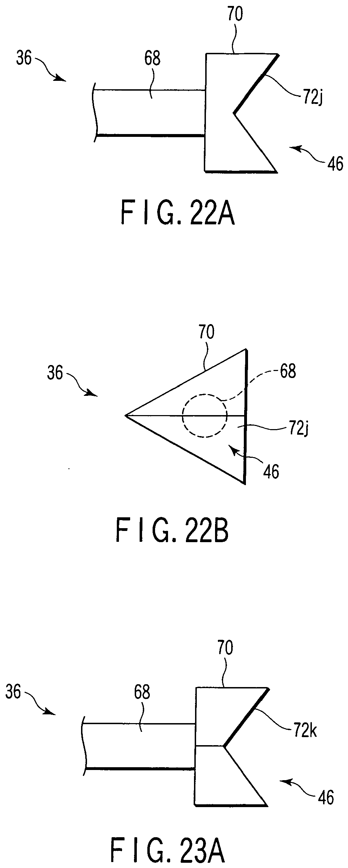

As shown in FIGS. 22A and 22B, a thick portion 70 according to a tenth modification has a substantially short triangular prism shape whose central axis is coaxial with a thin portion 68. A concave portion 72j is formed on an entire distal end surface of the thick portion 70, and this concave portion 72j has a base bottom portion having a substantially linear shape which is substantially orthogonal to a central axis of the thin portion 68 and runs substantially through an apex angle portion of the triangular prism shape. Furthermore, a cross section of the concave portion 72j perpendicular to the base bottom portion has a substantially triangular shape which opens toward the distal end side.

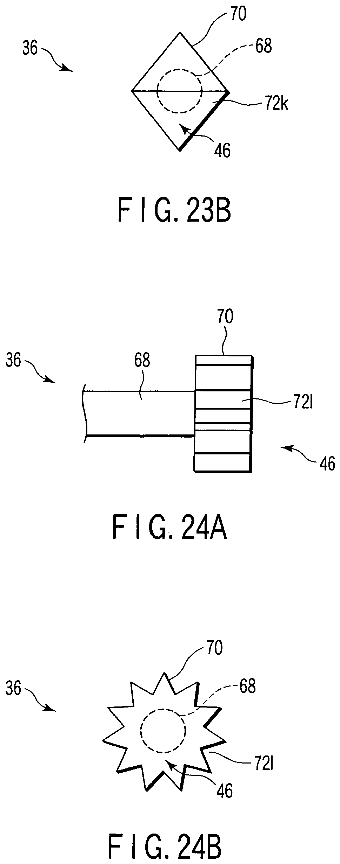

As shown in FIGS. 23A and 23B, a thick portion 70 according to an eleventh modification has a substantially short rhombic prism shape whose central axis is coaxial with a thin portion 68. A concave portion 72k is formed on an entire distal end surface of the thick portion 70, and this concave portion 72k has a base bottom portion having a substantially linear shape which is substantially orthogonal to a central axis of the thin portion 68 and runs substantially through both opposed apex angle portions of the rhombic prism shape. Moreover, a cross section of the concave portion 72k perpendicular to the base bottom portion has a substantially triangular shape which opens toward an distal end side.

As shown in FIGS. 24A and 24B, a thick portion 70 according to a twelfth modification has a substantially short polygon prism shape whose central axis is coaxial with a thin portion 58 and which has a plurality of radial protruding portions extending in a radial direction. Each concave portion 721 is formed between the plurality of radial protruding portions on an distal end surface of the thick portion 70.

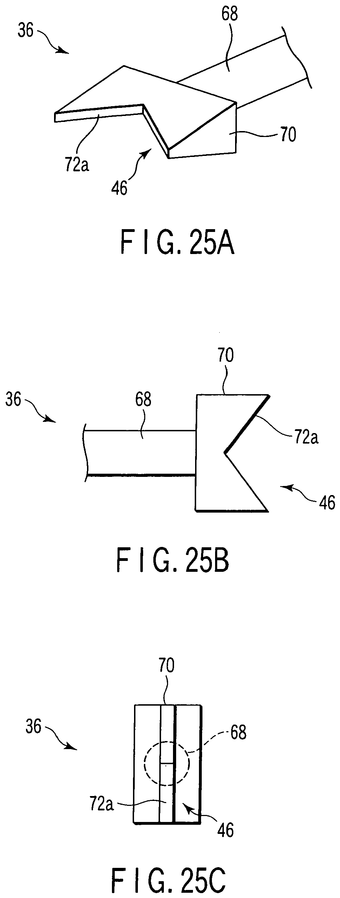

As shown in FIGS. 25A to 25C, a thick portion 70 according to a thirteenth modification has a configuration in which a length of each short side of a substantially rectangular shape of a cross section perpendicular to a central axis of a thin portion 68 is continuously changed in such a manner that it becomes longer than a diameter of the thin portion 68 on a proximal end surface and shorter than the same on an distal end surface in the thick portion 70 according to the ninth embodiment.

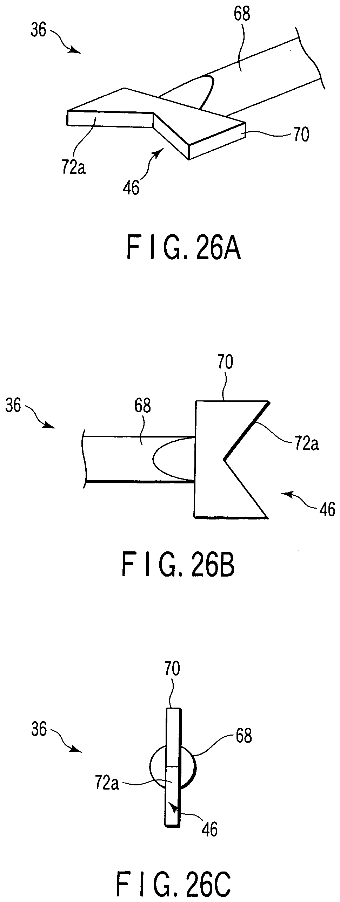

As shown in FIGS. 26A to 26C, a thick portion 70 according to a fourteenth modification has a configuration in which a length of each short side of a substantially rectangular shape of a cross section perpendicular to a central axis of a thin portion 68 is set smaller than a diameter of the thin portion 68 in the thick portion 70 according to the ninth embodiment.

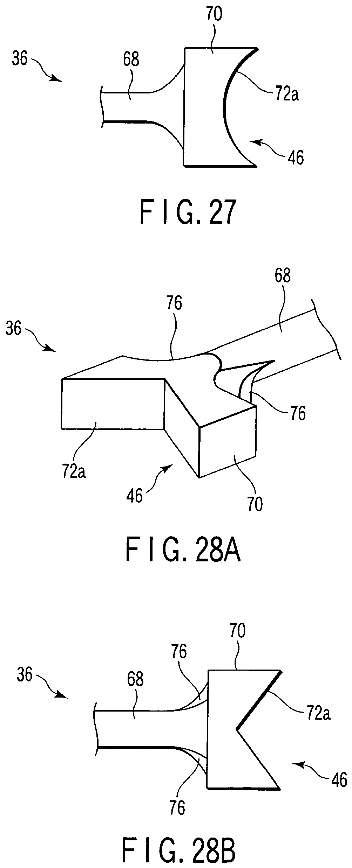

As shown in FIG. 27, in a fifteenth modification, an R chamfer or a C chamfer is formed at a connecting portion between a thin portion 68 and a thick portion 70 in order to improve strength.

FIGS. 28A and 28B show a tenth embodiment according to the present invention. Like reference numbers denote structures having the same functions as those in the ninth embodiment, thereby eliminating their explanation. In this embodiment, a knife-shaped edge portion 76 is extended at a connecting portion between a thin portion 68 and a thick portion 70 in a longitudinal direction of the thin portion 68. In case of performing an incision treatment with respect to a treatment target, a treatment portion 36 is caused to perform ultrasonic vibrations to longitudinally vibrate the edge portion 76 in its extending direction, and a living tissue is caught by the edge portion 76, thereby effecting the incision treatment. In this embodiment, the incision treatment is performed with respect to the treatment target by using the knife-shaped edge portion 76, and hence incision efficiency is improved.

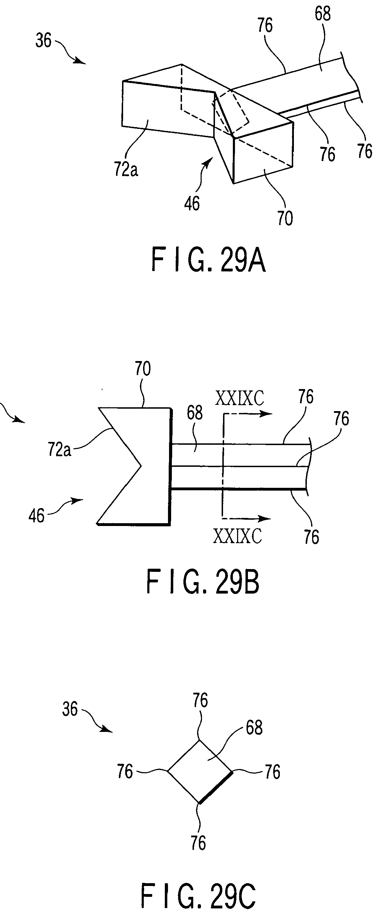

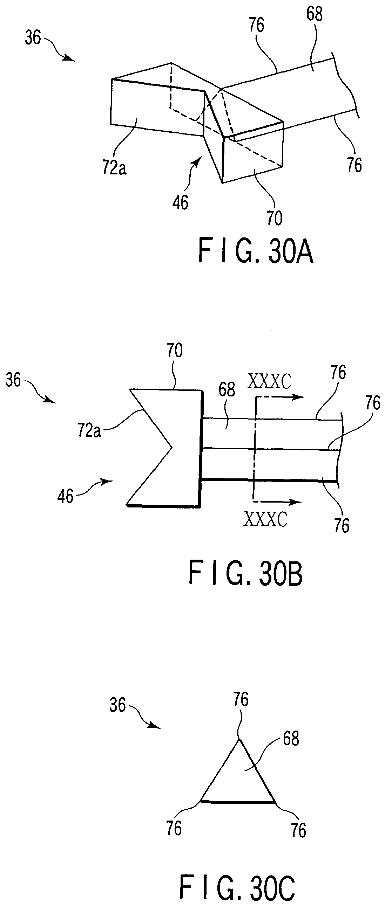

FIGS. 29A to 30C show first and second modifications of the tenth embodiment according to the present invention. As shown in FIGS. 29A to 29C, a cross section of a thin portion 68 according to the first modification perpendicular to its central axis has a substantially rhombic shape. Additionally, first to fourth edge portions 76 are provided to the thin portion 68 to extend in an axial direction of the thin portion 68 in accordance with respective apexes of this rhombic shape. Further, as shown in FIGS. 30A to 30C, a cross section of a thin portion 68 according to the second modification perpendicular to its central axis has a substantially triangular shape. Furthermore, first to third edge portions 76 are provided to the thin portion 68 to extend in an axial direction of the thin portion 68 in accordance with respective apexes of this triangular shape. In case of performing an incision treatment with respect to a treatment target by using an ultrasonic treatment apparatus 12 according to the first or second modification, a treatment portion 36 is caused to perform ultrasonic vibrations to longitudinally vibrate the edge portions 76 in the extending direction thereof, and the edge portions 76 are pressed against a living tissue, thereby effecting the incision treatment.

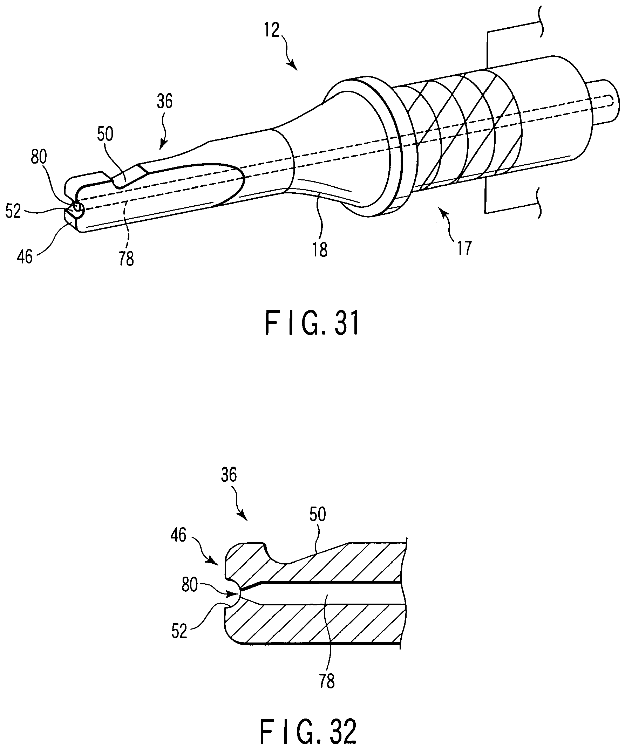

FIG. 31 shows an eleventh embodiment according to the present invention. Like reference numbers denote structures having the same functions as those in the eighth embodiment, thereby eliminating their explanation. A channel 78 extending in a proximal end side toward an distal end side is formed in a treatment portion 36 and an ultrasonic transducer 17 according to this embodiment. An distal end portion of this channel 78 opens at a base bottom portion of a concave portion 52 on an distal end surface portion 46 of the treatment portion 36, thereby forming an distal end opening portion 80. On the other hand, a proximal end portion of the channel 78 communicates with a connecter arranged in a main body portion 16 (see FIG. 1A) which accommodates the ultrasonic transducer 17, and this connecter is connected with a suction device, a solution sending device or the like through, e.g., a tube.

A function of an ultrasonic treatment apparatus 12 according to this embodiment will now be described. A description will be given as to a case where a tissue in which fibers are mixed is fractured by cavitation to perform a treatment like the ninth embodiment. When giving a treatment to the fibers by using the treatment portion 36, the ultrasonic treatment apparatus 12 is connected with a suction device as required, the suction device is operated to perform suction from the distal end opening portion 80, and the fibers are pulled into the concave portion 52 and the treatment is performed using the distal end surface portion 46. Further, fractured tissues generated from the treatment are collected from the distal end opening portion 80. Furthermore, the ultrasonic treatment apparatus 12 is connected with the solution sending device, the solution sending device is operated to supply a physiological saline from the distal end opening portion, and the treatment is given to the fibers in a state where the treatment portion 36 is dipped in the physiological saline. As a result, large cavitation is generated to fracture the fibers. Moreover, the solution sending device is operated to supply a homeostatic agent such as ethanol from the distal end opening portion 80 to a bleeding site, thereby stopping bleeding at the bleeding site.

Therefore, the ultrasonic treatment apparatus 12 according to this embodiment includes the following effect. In this embodiment, in case of performing a treatment by using the distal end surface portion 46 of the treatment portion 36, suction/supply of a solution can be performed from the distal end opening portion 80 formed at the base bottom portion of the concave portion 52 on the distal end surface portion 46. That is, combining the treatment using the distal end surface portion 46 with suction/supply of a solution from the distal end opening portion 80 can effect an optimum treatment with respect to a treatment target.

FIG. 32 shows a twelfth embodiment according to the present invention. Like reference numbers denote structures having the same functions as those in the eleventh embodiment, thereby eliminating their explanation. In a treatment portion 36 according to this embodiment, a diameter of a channel 78 in the distal end opening portion 80 is smaller than that of a channel 78 on a proximal end side of the distal end opening portion 80. In case of giving a treatment by using an distal end surface portion 46 of the treatment portion 36, the treatment is given to a treatment target by using a part other than the distal end opening portion 80 on the distal end surface portion 46. In this embodiment, since the diameter of the channel 78 is small in the distal end opening portion 80, a part of the distal end surface portion 46 which gives the treatment to the treatment target is large, thereby improving a treatment capability.

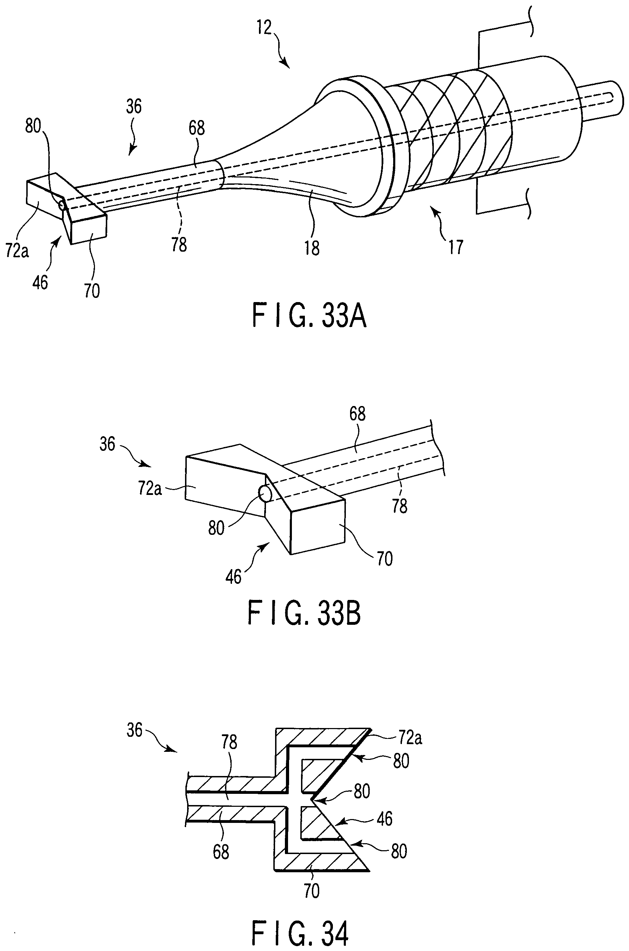

FIGS. 33A and 33B show a thirteenth embodiment according to the present invention. Like reference numbers denote structures having the same functions as those in the ninth embodiment, thereby eliminating their explanation. In this embodiment, a channel 78, an distal end opening portion 80 and a connecter are formed in the ultrasonic treatment apparatus 12 according to the ninth embodiment, like the tenth embodiment. Therefore, the same functions and effects as those in the tenth embodiment are demonstrated.

FIG. 34 shows a fourteenth embodiment according to the present invention. Like reference numbers denote structures having the same functions as those in the thirteenth embodiment, thereby eliminating their explanation. In this modification, an distal end portion of a channel 78 is branched, whereby a plurality of distal end opening portions 80 are formed on an distal end surface portion 46. In case of performing suction/supply of a solution, suction/supply of a solution is performed from the plurality of distal end opening portions 80 on the distal end surface portion 46. As described above, in this embodiment, since suction/supply of a solution is carried out from the plurality of distal end opening portions 80 on the distal end surface portion 46, suction/supply of a solution can be effected on the entire distal end surface portion 46.

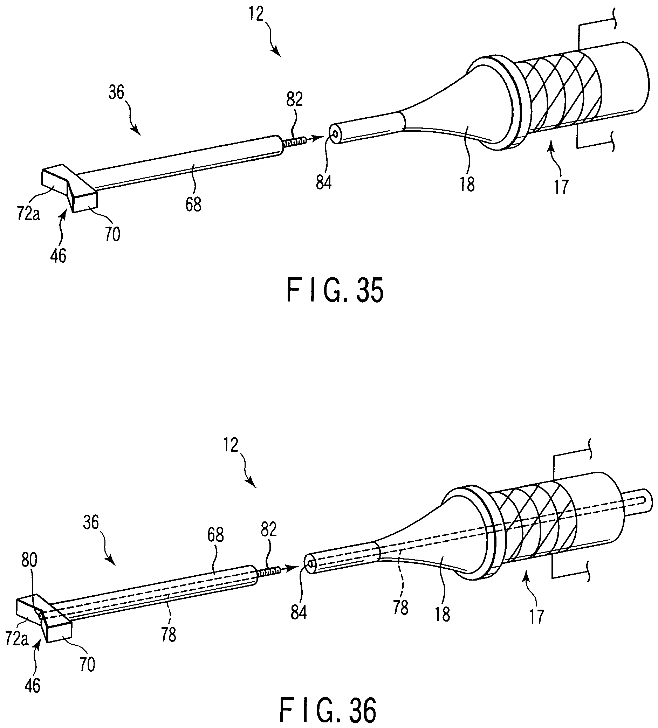

FIG. 35 shows a fifteenth embodiment according to the present invention. Like reference numbers denote structures having the same functions as those in the ninth embodiment, thereby eliminating their explanation. This embodiment has a structure in which a treatment portion 36 can be freely attached to/detached from an ultrasonic transducer 17 in the ninth embodiment. That is, a male screw portion 82 is extended at a proximal end portion of a thin portion 68 of a treatment portion 36 in a central axis direction of the thin portion 68, and a female screw portion 84 corresponding to the male screw portion 82 of the thin portion 68 is bored at an output end 18 of the ultrasonic transducer 17 in a vibration direction. Furthermore, the male screw portion 82 of the thin portion 68 is detachably screwed in the female screw portion 84 of the ultrasonic transducer 17. When the treatment portion 36 is worn out by continuously using the ultrasonic treatment apparatus 12, the treatment portion 36 is removed from the ultrasonic transducer 17, and a new treatment portion 36 is attached. The treatment portion 36 is most apt to be worn in the ultrasonic treatment apparatus 12. In this embodiment, when the treatment portion 36 is worn out, the treatment portion 36 can be replaced, thereby extending a life duration of the entire ultrasonic treatment apparatus 12.

FIG. 36 shows a modification of the fifteenth embodiment according to the present invention. Like reference numbers denote structures having the same function as those in the thirteenth embodiment, thereby eliminating their explanation. This embodiment has a structure in which a treatment portion 36 can be freely attached to/detached from an ultrasonic transducer 17 in the thirteenth embodiment. That is, a male screw portion 82 is extended at a proximal end portion of a thin portion 38 in a central axis direction of the thin portion 68, and a channel 78 penetrates the male screw portion 82 to be opened on a proximal end surface of the male screw portion 82. On the other hand, the channel 78 is opened on an distal end surface of an output terminal 18 of the ultrasonic transducer 17, and a female screw portion 84 corresponding to the male screw portion 82 of the thin portion 68 is extended at an distal end portion of the channel 78 of the ultrasonic transducer 17 in a vibration direction. Furthermore, the male screw portion 82 of the thin portion 68 is detachably screwed in the female screw portion 84 of the ultrasonic transducer 17, whereby the channel 78 of the ultrasonic transducer 17 communicates with the channel 78 of the treatment apparatus.

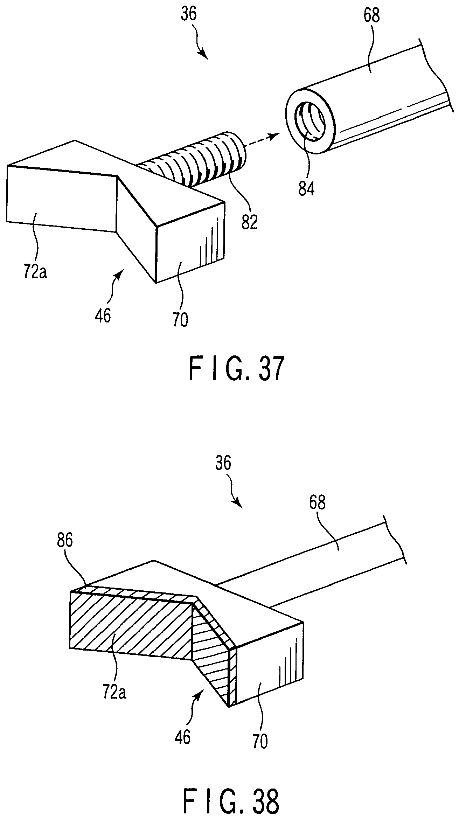

FIG. 37 shows a sixteenth embodiment according to the present invention. Like reference numbers denote structures having the same functions as those in the ninth embodiment, thereby eliminating their explanation. This embodiment has a structure in which a thick portion 70 is attachable/detachable with respect to a thin portion 68 and the thick portion 70 is formed of highly durable ceramics in the ninth embodiment. That is, a male screw portion 82 is provided to protrude on a proximal end surface of the thick portion 70 of a treatment portion 36 in a direction substantially perpendicular to the proximal end surface, and a female screw portion 84 corresponding to the male screw portion 82 of the thick portion 70 is bored at the distal end portion of the thin portion 68 along a central axis direction of the thin portion 68. Additionally, the male screw portion 82 of the thick portion 70 is detachably screwed in the female screw portion 84 of the thin portion 68. Further, the thick portion 70 is formed of ceramics such as alumina or zirconia.

In case of giving a treatment by using the ultrasonic treatment apparatus 12, the treatment is performed by mainly using the thick portion 70 in the treatment portion 36. Further, when the thick portion 70 is worn out by continuously using the ultrasonic treatment apparatus 12, the thick portion 70 is removed from the thin portion 68, and a new thick portion 70 is attached. The thick portion 70 is most apt to be worn in the ultrasonic treatment apparatus 12. In this embodiment, this thick portion 70 is formed of highly durable ceramics so that it is hard to be worn out, and the thick portion 70 can be replaced when the thick portion 70 is worn out. Therefore, a life duration of the entire ultrasonic treatment apparatus 12 is extended.

FIG. 38 shows a modification of the sixteenth embodiment according to the present invention. Like reference numbers denote structures having the same functions as those in the sixteenth embodiment, thereby eliminating their explanation. In this embodiment, an distal end surface portion 46 alone in a thick portion 70 of a treatment portion 36 is formed of ceramics. That is, a sheet-like durable member 86 formed of ceramics such as alumina or zirconia is brazed on an distal end surface of the thick portion 70 of the treatment portion 36.

### Industrial Applicability

The present invention provides the ultrasonic treatment apparatus, which applies ultrasonic vibrations to a treatment target to perform a treatment, capable of preventing the slipping of the treatment target to certainly perform the treatment.

## Claims

1. An ultrasonic treatment apparatus comprising:
an ultrasonic transducer (17) configured to generate an ultrasonic vibration; and
a treatment portion (36) including a proximal end portion, configured to transmit the ultrasonic vibration generated by the ultrasonic transducer (17) in an axial direction and apply the ultrasonic vibration to a treatment target, and including a distal end surface (46) **characterised in that**
said distal end surface (46) is facing in the axial direction, configured to be brought into contact with the treatment target, and includes a concave holding portion (52; 53a; 53b; 54; 57; 72a; 72b; 72c; 72d; 73d; 72e; 72f; 729; 72h; 72i; 72j; 72k) formed on the distal end surface (46) of the treatment portion (36) and configured to hold the treatment target in contact with the distal end surface (46) of the treatment portion (36).

2. The ultrasonic treatment apparatus according to claim 1, **characterized in that** the proximal end portion of the treatment portion (36) is connected to the ultrasonic transducer (17).

3. The ultrasonic treatment apparatus according to claim 1, **characterized by** further comprising an elongated vibration transmitting portion (28) including a proximal end portion connected to the ultrasonic transducer (17) and a distal end portion connected to the proximal end portion of the treatment portion (36).

4. The ultrasonic treatment apparatus according to claim 2, **characterized in that** the ultrasonic treatment apparatus further comprises an indicator (64a; 64b; 64c; 64d; 64e; 64f) indicating characteristics of the holding portion (52; 53a; 53b; 54; 57; 72a; 72b; 72c; 72d; 73d; 72e; 72f; 72g; 72h; 72i; 72j; 72k).

5. The ultrasonic treatment apparatus according to claim 4, **characterized in that** the treatment portion (36) includes a peripheral surface (48), and the indicator (64a; 64b; 64c; 64d; 64e; 64f) is formed on the peripheral surface (48) of the treatment portion (36).

6. The ultrasonic treatment apparatus according to claim 3, **characterized in that** the ultrasonic treatment apparatus further comprises an indicator (64a; 64b; 64c; 64d; 64e; 64f) indicating characteristics of the holding portion (52; 53a; 53b; 54; 57; 72a; 72b; 72c; 72d; 73d; 72e; 72f; 72g; 72h; 72i; 72j; 72k).

7. The ultrasonic treatment apparatus according to claim 6, **characterized in that** the treatment portion (36) includes a peripheral surface (48), the vibration transmitting portion (28) includes a peripheral surface, and the indicator (64a; 64b; 64c; 64d; 64e; 64f) is formed on the peripheral surface (48) of the treatment portion (36) or the peripheral surface of the vibration transmitting portion (28).

8. The ultrasonic treatment apparatus according to claim 4 or 6, **characterized in that** the characteristics of the holding portion (52; 53a; 53b; 54; 57; 72a; 72b; 72c; 72d; 73d; 72e; 72f; 72g; 72h; 72i; 72j; 72k) include an arrangement or a type of the holding portion (52; 53a: 53b; 54; 57; 72a; 72b; 72c; 72d; 73d; 72e; 72f; 72g; 72h; 72i; 72j; 72k).

9. The ultrasonic treatment apparatus according to claim 2, **characterized in that** the treatment portion (36) includes a channel (78) penetrating the treatment portion (36) from a proximal end side to a distal end side and including an opening portion (80) on the distal end surface (46) of the treatment portion (36).

10. The ultrasonic treatment apparatus according to claim 3, **characterized in that** the vibration transmitting portion (28) and the treatment portion (36) include a channel (78) penetrating the vibration transmitting portion (28) and the treatment portion (36) from a proximal end side to a distal end side and including an opening portion (80) on the distal end surface (46) of the treatment portion (36).

11. The ultrasonic treatment apparatus according to claim 9 or 10, **characterized in that** a diameter of the channel (78) in the opening portion (80) is smaller than that of the channel (78) on the proximal end side of the opening portion (80).

12. The ultrasonic treatment apparatus according to claim 2 or 3, **characterized in that** the treatment portion (36) includes a thin portion (68) extending from a proximal end side to a distal end side and a thick portion (70) provided at a distal end portion of the thin portion (68), and an external diameter of at least a part of a cross section of the thick portion (70) perpendicular to a longitudinal axis of the thin portion (68) is larger than an external diameter of at least a part of a cross section of the thin portion (68) perpendicular to the longitudinal axis of the thin portion (68).

13. The ultrasonic treatment apparatus according to claim 12, **characterized in that** the treatment portion (36) includes a knife-shaped edge portion (76) provided to the thin portion (68) or between the thin portion (68) and the thick portion (70).

14. The ultrasonic treatment apparatus according to claim 3, **characterized in that** the treatment portion (36) is detachably connected to the vibration transmitting portion (28).

15. The ultrasonic treatment apparatus according to claim 12, **characterized in that** the thick portion (70) is detachably connected to the thin portion (68).

16. The ultrasonic treatment apparatus according to claim 2 or 3, **characterized in that** the distal end surface (46) of the treatment portion (36) is formed of ceramics.

## Patentansprüche

1. Ultraschallbehandlungsgerät, umfassend:
einen Ultraschallwandler (17), der konfiguriert ist, um eine Ultraschallvibration zu generieren; und
einen Behandlungsabschnitt (36), der einen proximalen Endabschnitt umfasst, der konfiguriert ist, um die Ultraschallvibration, die von dem Ultraschallwandler (17) generiert wird, in einer Axialrichtung zu übertragen und die Ultraschallvibration auf ein Behandlungsziel anzuwenden, und der eine distale Endfläche (46) umfasst,
**dadurch gekennzeichnet, dass** die distale Endfläche (46) der Axialrichtung zugewandt ist, konfiguriert ist, um mit dem Behandlungsziel in Kontakt gebracht zu werden, und einen konkaven Halteabschnitt (52; 53a, 53b, 57, 72a, 72b; 72c; 72d; 73d; 72e; 72f; 72g; 72h; 72i; 72j; 72k) umfasst, der auf der distalen Endfläche (46) des Behandlungsabschnitts (36) gebildet ist und konfiguriert ist, um das Behandlungsziel in Kontakt mit der distalen Endfläche (46) des Behandlungsabschnitts (36) zu halten.

2. Ultraschallbehandlungsgerät nach Anspruch 1, **dadurch gekennzeichnet, dass** der proximale Endabschnitt des Behandlungsabschnitts (36) mit dem Ultraschallwandler (17) verbunden ist.

3. Ultraschallbehandlungsgerät nach Anspruch 1, **dadurch gekennzeichnet, dass** es ferner einen länglichen Vibrationsübertragungsabschnitt (28) umfasst, der einen proximalen Endabschnitt, der mit dem Ultraschallwandler (17) verbunden ist, und einen distalen Endabschnitt, der mit dem proximalen Endabschnitt des Behandlungsabschnitts (36) verbunden ist, umfasst.

4. Ultraschallbehandlungsgerät nach Anspruch 2, **dadurch gekennzeichnet, dass** das Ultraschallbehandlungsgerät ferner einen Indikator (64a; 64b; 64c; 64d; 64e; 64f) umfasst, der die Merkmale des Halteabschnitts (52; 53a; 53b; 54; 57; 72a; 72b; 72c; 72d; 73d; 72e; 72f; 72g; 72h; 72i; 72j; 72k) angibt.

5. Ultraschallbehandlungsgerät nach Anspruch 4, **dadurch gekennzeichnet, dass** der Behandlungsabschnitt (36) eine periphere Oberfläche (48) umfasst, und der Indikator (64a; 64b; 64c; 64d; 64e; 64f) auf der peripheren Oberfläche (48) des Behandlungsabschnitts (36) gebildet ist.

6. Ultraschallbehandlungsgerät nach Anspruch 3, **dadurch gekennzeichnet, dass** das Ultraschallbehandlungsgerät ferner einen Indikator (64a; 64b; 64c; 64d; 64e; 64f) umfasst, der die Merkmale des Halteabschnitts (52; 53a; 53b; 54; 57; 72a; 72b; 72c; 72d; 73d; 72e; 72f; 72g; 72h; 72i; 72j; 72k) angibt.

7. Ultraschallbehandlungsgerät nach Anspruch 6, **dadurch gekennzeichnet, dass** der Behandlungsabschnitt (36) eine periphere Oberfläche (48) umfasst, der Vibrationsübertragungsabschnitt (28) eine periphere Oberfläche umfasst, und der Indikator (64a; 64b; 64c; 64d; 64e; 64f) auf der peripheren Oberfläche (48) des Behandlungsabschnitts (36) oder der peripheren Oberfläche des Vibrationsübertragungsabschnitts (28) gebildet ist.

8. Ultraschallbehandlungsgerät nach Anspruch 4 oder 6, **dadurch gekennzeichnet, dass** die Merkmale des Halteabschnitts (52; 53a; 53b; 54; 57; 72a; 72b; 72c; 72d; 73d; 72e; 72f; 72g; 72h; 72i; 72j; 72k) eine Anordnung oder eine Art des Halteabschnitts (52; 53a; 53b; 54; 57; 72a; 72b; 72c; 72d; 73d; 72e; 72f; 72g; 72h; 72i; 72j; 72k) umfassen.

9. Ultraschallbehandlungsgerät nach Anspruch 2, **dadurch gekennzeichnet, dass** der Behandlungsabschnitt (36) einen Kanal (78) umfasst, der von einer proximalen Endseite zu einer distalen Endseite in den Behandlungsabschnitt (36) eindringt und einen Öffnungsabschnitt (80) auf der distalen Endfläche (46) des Behandlungsabschnitts (36) umfasst.

10. Ultraschallbehandlungsgerät nach Anspruch 3, **dadurch gekennzeichnet, dass** der Vibrationsübertragungsabschnitt (28) und der Behandlungsabschnitt (36) einen Kanal (78) umfassen, der von einer proximalen Endseite zu einer distalen Endseite in den Vibrationsübertragungsabschnitt (28) und den Behandlungsabschnitt (36) eindringt und einen Öffnungsabschnitt (80) auf der distalen Endfläche (46) des Behandlungsabschnitts (36) umfasst.

11. Ultraschallbehandlungsgerät nach Anspruch 9 oder 10, **dadurch gekennzeichnet, dass** ein Durchmesser des Kanals (78) in dem Öffnungsabschnitt (80) kleiner als der des Kanals (78) auf der proximalen Endseite des Öffnungsabschnitts (80) ist.

12. Ultraschallbehandlungsgerät nach Anspruch 2 oder 3, **dadurch gekennzeichnet, dass** der Behandlungsabschnitt (36) einen dünnen Abschnitt (68), der sich von einer proximalen Endseite bis zu einer distalen Endseite erstreckt, und einen dicken Abschnitt (70), der an einem distalen Endabschnitt des dünnen Abschnitts (68) bereitgestellt wird, umfasst, und ein externer Durchmesser mindestens eines Teils eines Querschnitts des dicken Abschnitts (70) rechtwinklig zu einer Längsachse des dünnen Abschnitts (68) größer als ein externer Durchmesser mindestens eines Teils eines Querschnitts des dünnen Abschnitts (68) rechtwinklig zur Längsachse des dünnen Abschnitts (68) ist.

13. Ultraschallbehandlungsgerät nach Anspruch 12, **dadurch gekennzeichnet, dass** der Behandlungsabschnitt (36) einen messerförmigen Randabschnitt (76) umfasst, der an dem dünnen Abschnitt (68) oder zwischen dem dünnen Abschnitt (68) und dem dicken Abschnitt (70) bereitgestellt wird.

14. Ultraschallbehandlungsgerät nach Anspruch 3, **dadurch gekennzeichnet, dass** der Behandlungsabschnitt (36) abnehmbar mit dem Vibrationsübertragungsabschnitt (28) verbunden ist.

15. Ultraschallbehandlungsgerät nach Anspruch 12, **dadurch gekennzeichnet, dass** der dicke Abschnitt (70) mit dem dünnen Abschnitt (68) abnehmbar verbunden ist.

16. Ultraschallbehandlungsgerät nach Anspruch 2 oder 3, **dadurch gekennzeichnet, dass** die distale Endfläche (46) des Behandlungsabschnitts (36) aus Keramik besteht.

## Revendications

1. Appareil de traitement ultrasonique comprenant :
un transducteur ultrasonique (17) configuré pour générer une vibration ultrasonique ; et
une partie (36) de traitement incluant une partie d'extrémité proximale, configurée pour transmettre la vibration ultrasonique générée par le transducteur ultrasonique (17) dans une direction axiale et appliquer la vibration ultrasonique à une cible de traitement, et incluant une surface (46) d'extrémité distale, **caractérisé en ce que** ladite surface (46) d'extrémité distale fait face à la direction axiale, configurée pour être mise en contact avec la cible de traitement, et inclut une partie de maintien (52 ; 53a ; 53b ; 54 ; 57 ; 72a ; 72b ; 72c ; 72d ; 73d ; 72e ; 72f ; 729 ; 72h ; 72i ; 72j ; 72k) concave formée sur la surface (46) d'extrémité distale de la partie (36) de traitement et configurée pour maintenir la cible de traitement en contact avec la surface (46) d'extrémité distale de la partie (36) de traitement.

2. Appareil de traitement ultrasonique selon la revendication 1, **caractérisé en ce que** la partie d'extrémité proximale de la partie (36) de traitement est connectée au transducteur ultrasonique (17).

3. Appareil de traitement ultrasonique selon la revendication 1, **caractérisé en ce que** comprenant en outre une partie (28) allongée de transmission de vibration incluant une partie d'extrémité proximale connectée au transducteur ultrasonique (17) et une partie d'extrémité distale connectée à la partie d'extrémité proximale de la partie (36) de traitement.

4. Appareil de traitement ultrasonique selon la revendication 2, **caractérisé en ce que** l'appareil de traitement ultrasonique comprend en outre un indicateur (64a ; 64b ; 64c ; 64d ; 64e ; 64f) indiquant des caractéristiques de la partie de maintien (52 ; 53a ; 53b ; 54 ; 57 ; 72a ; 72b ; 72c ; 72d ; 73d ; 72e ; 72f ; 72g ; 72h ; 72i ; 72j ; 72k).

5. Appareil de traitement ultrasonique selon la revendication 4, **caractérisé en ce que** la partie (36) de traitement inclut une surface périphérique (48), et l'indicateur (64a ; 64b ; 64c ; 64d ; 64e ; 64f) est formé sur la surface périphérique (48) de la partie (36) de traitement.

6. Appareil de traitement ultrasonique selon la revendication 3, **caractérisé en ce que** l'appareil de traitement ultrasonique comprend en outre un indicateur (64a ; 64b ; 64c ; 64d ; 64e ; 64f) indiquant des caractéristiques de la partie de maintien (52 ; 53a ; 53b ; 54 ; 57 ; 72a ; 72b ; 72c ; 72d ; 73d ; 72e ; 72f ; 72g ; 72h ; 72i ; 72j ; 72k).

7. Appareil de traitement ultrasonique selon la revendication 6, **caractérisé en ce que** la partie (36) de traitement inclut une surface périphérique (48), la partie (28) de transmission de vibration inclut une surface périphérique, et l'indicateur (64a ; 64b ; 64c ; 64d ; 64e ; 64f) est formé sur la surface périphérique (48) de la partie (36) de traitement ou la surface périphérique de la partie (28) de transmission de vibration.

8. Appareil de traitement ultrasonique selon la revendication 4 ou 6, **caractérisé en ce que** les caractéristiques de la partie de maintien (52 ; 53a ; 53b ; 54 ; 57 ; 72a ; 72b ; 72c ; 72d ; 73d ; 72e ; 72f ; 72g ; 72h ; 72i ; 72j ; 72k) incluent un agencement ou un type de la partie de maintien (52 ; 53a ; 53b ; 54 ; 57 ; 72a ; 72b ; 72c ; 72d ; 73d ; 72e ; 72f ; 72g ; 72h ; 72i ; 72j ; 72k).

9. Appareil de traitement ultrasonique selon la revendication 2, **caractérisé en ce que** la partie (36) de traitement inclut un canal (78) pénétrant la partie (36) de traitement d'un côté d'extrémité proximale à un côté d'extrémité distale et incluant une partie (80) d'ouverture sur la surface (46) d'extrémité distale de la partie (36) de traitement.

10. Appareil de traitement ultrasonique selon la revendication 3, **caractérisé en ce que** la partie (28) de transmission de vibration et la partie (36) de traitement incluent un canal (78) pénétrant la partie (28) de transmission de vibration et la partie (36) de traitement d'un côté d'extrémité proximale à un côté d'extrémité distale et incluant une partie (80) d'ouverture sur la surface (46) d'extrémité distale de la partie (36) de traitement.

11. Appareil de traitement ultrasonique selon la revendication 9 ou 10, **caractérisé en ce qu'**un diamètre du canal (78) dans la partie (80) d'ouverture est plus petit que celui du canal (78) sur le côté d'extrémité proximale de la partie (80) d'ouverture.

12. Appareil de traitement ultrasonique selon la revendication 2 ou 3, **caractérisé en ce que** la partie (36) de traitement inclut une partie mince (68) s'étendant d'un côté d'extrémité proximale à un côté d'extrémité distale et une partie épaisse (70) prévue au niveau d'une partie d'extrémité distale de la partie mince (68), et un diamètre externe d'au moins une partie d'une section transversale de la partie épaisse (70) perpendiculaire à un axe longitudinal de la partie mince (68) est plus grand qu'un diamètre externe d'au moins une partie d'une section transversale de la partie mince (68) perpendiculaire à l'axe longitudinal de la partie mince (68).

13. Appareil de traitement ultrasonique selon la revendication 12, **caractérisé en ce que** la partie (36) de traitement inclut une partie (76) de bord en forme de couteau prévue sur la partie mince (68) ou entre la partie mince (68) et la partie épaisse (70).

14. Appareil de traitement ultrasonique selon la revendication 3, **caractérisé en ce que** la partie (36) de traitement est connectée de manière détachable à la partie (28) de transmission de vibration.

15. Appareil de traitement ultrasonique selon la revendication 12, **caractérisé en ce que** la partie épaisse (70) est connectée de manière détachable à la partie mince (68).

16. Appareil de traitement ultrasonique selon la revendication 2 ou 3, **caractérisé en ce que** la surface (46) d'extrémité distale de la partie (36) de traitement est constituée d'une céramique.
